(19) Europäisches Patentamt

European Patent Office

Office européen des brevets

(11) Publication number: **0 319 551 B1**

# EUROPEAN PATENT SPECIFICATION

(45) Date of publication of patent specification: **16.03.94**

(51) Int. Cl.5: **A61B 5/02, A61B 5/00**

(21) Application number: **88900299.4**

(22) Date of filing: **04.12.87**

(86) International application number:
**PCT/US87/03116**

(87) International publication number:
**WO 86/09146 (01.12.88 26/88)**

(54) **PERSONAL MONITOR AND PROCESS FOR HEAT AND WORK STRESS.**

(30) Priority: **29.05.87 US 55654**

(43) Date of publication of application:
**14.06.89 Bulletin 89/24**

(45) Publication of the grant of the patent:
**16.03.94 Bulletin 94/11**

(84) Designated Contracting States:
**AT BE CH DE FR GB IT LI LU NL SE**

(56) References cited:
**EP-A- 0 117 330        DE-A- 2 913 048
US-A- 3 978 849        US-A- 4 090 504
US-A- 4 117 834        US-A- 4 129 125
US-A- 4 312 358**

**Journal A, vol.24, no.4, October 1983 (Antwerpen BE) M.Woerlee: "Measurements of human work and work environment", pages 195-204**

(73) Proprietor: **ELECTRIC POWER RESEARCH INSTITUTE, INC
3412 Hillview Avenue
Palo Alto California 94303(US)**

(72) Inventor: **BERNARD, Thomas, E.
1118 Varner Road
Pittsburgh, PA 15227(US)**
Inventor: **SHERWIN, Gary W.**

**Yukon, PA 15698(US)**
Inventor: **KENNEY, William, L., Jr.
211 Mary Elizabeth Street
Boalsburg, PA 16827(US)**
Inventor: **LEWIS, Debra, E.
210 East Hamilton Avenue, No. 27
State College, PA 16801(US)**

(74) Representative: **Jackson, David Spence et al
REDDIE & GROSE
16, Theobalds Road
London, WC1X 8PL (GB)**

EP 0 319 551 B1

## Description

The present invention relates to a personal monitor of the kind which comprises a heart beat sensor producing output electrical signals indicating a user's heart beats, means for storing heart beat information corresponding to the heart beat signals produced, information processing means connected to receive the heart beat information from said storing means, said information processing means being configured to analyse the heart beat information to obtain a physiological demand and to compare the physiological demand against a stored physiological demand limit, and means for providing an indication to the user when the stored physiological demand limit has been exceeded, said information processing means being configured and connected to control operation of said indication providing means. The invention also relates to monitoring processes.

Two approaches are conventional to avoid excessive heart rates and increases in body temperature during heat stress exposures. One is to limit the exposure through administrative controls, such as stay times. The other is to use self-determination. Stay times are usually very conservative to protect most people. Self-determination often leads an individual to overextend himself or herself.

A variety of measuring devices are known in the art for measuring exertion and related parameters. Body core temperature is a basic physiological measure of the body's response to heat stress. Rectal temperature is commonly accepted as a primary measure. Because rectal temperature is measured by an inserted probe, it is not practical or acceptable for routine evaluation of core temperature. A substitute measure is required.

Commonly used substitutes for rectal temperature are ear, esophageal, and oral temperatures. While the temperature measuring technology for these is readily available, they pose many practical problems that make them unsuitable for a personal monitor. Swallowable or ingested thermotransmitters are possible, but unsuitable because of cost, potential liability and questionable acceptability.

A commercially available device can predict body core temperature by equalizing the heat flux from the core to the surface by placing a heater on the outside. However, this device requires too much power to be fully practical in this application, and it would give erroneous results in high ambient temperatures.

Heart rate is another physiological measure that is related to work and heat stress. There are several commercially available devices to measure heart rate in work environments. The primary markets for these devices are sport, exercise and rehabilitation. While the methods and embodiments vary somewhat, the primary method of monitoring is to provide a low and high threshold for heart rate, so that an alarm can be given if heart rate goes below or above the specified window.

For industrial environments, the low threshold does not have any real purpose. The high threshold is more valuable. If a person's heart rate exceeds the threshold, an alarm can warn him or her of possible overexertion. The problem with these high threshold warnings is how to set the threshold. Workers can and do have momentary peaks of high heart rates due to sudden bursts of activity or isometric work. These peaks do not represent sustained levels of work, but they result in an alarm that would be reasonable for sustained levels of high demand. However, with a simple high threshold, there would be many alarms that do not represent significant physiological strain. The situation is further complicated by prolonged work at an intermediate level. The high threshold would miss prolonged heart rates just below the threshold that are very significant physiological strains.

Examples of prior art devices for measuring heart rates, body temperature and related parameters are disclosed in the following issued U.S. Patents: 4,513,753, issued April 30, 1985 to Tabata et al.; 4,450,843, issued May 29, 1984 to Barney et al.; 4,425,921, issued January 17, 1984 to Fujisaki et al.; 4,409,985, issued October 18, 1983 to Sidorenko et al.; 4,378,111, issued March 29, 1983 to Tsuchida et al.; 4,367,752, issued January 11, 1983 to Jimenez et al.; 4,343,315, issued August 10, 1982 to O'Leary and 4,312,358, issued January 26, 1982 to Barney. The state of the art is further indicated by Humen, D.P. and Bougher, D.R., "Evaluation of Commercially Available Heart Rate Monitors," The Canadian Medical Association Journal, Vol. 131, September 15, 1984, pp. 585-589 and by commercially available heart rate monitors from Computer Instruments Corporation, Hempstead, L.I., N.Y. 11550; Biosig Instruments, Inc., Champlain, N.Y. 12919 and Dak Industries, Inc., Canoga Park, CA 91304.

United States patent no. 4,117,834 describes a physiological motor activity monitoring apparatus having a mercury switch which detects movements of the body of a person wearing the apparatus, a scaler arranged to scale the number of movements by dividing by a selectable factor, an accumulator that counts up the output of the scaler during each interval determined by resetting pulses from a frequency divider driven by a crystal oscillator, and a memory that stores the content of the accumulator at the end of each interval in a predetermined location of the memory. As all the memory locations become full, data is transferred out of these locations to an external permanent storage medium so that the monitoring apparatus can be reused and the data analysed. Thus the amount of motor activity is monitored and stored for many successive time intervals.

EP-A-0,117,330 describes a heart monitor of the kind defined hereinbefore at the beginning. In this known monitor, heart beat indications are provided as electrical pulses from a heart beat sensor and are coupled to a microprocessor with an internal memory. Part of the internal memory is used as a counter for clock pulses. The microprocessor calculates and stores the user's resting heart rate by counting up the pulses from the heart beat sensor for a prescribed period such as six seconds. The user then undergoes a submaximal exercise test during which the user's heart beat rate is measured and updated every six beats or every ten seconds. The maximal heart rate is calculated as 220-AGE (years). The exercise test ends when either the heart beat rate reaches the calculated maximal value or the user feels too exhausted to continue. The time taken to reach this condition is measured and the microprocessor calculates from the maximal heart rate or the heart rate at exhaustion and the time taken the maximal oxygen uptake of the user. The monitor can then be used for exercise monitoring by being set in a state in which it emits pacer tones at a rate chosen by the user. The user's heart rate is monitored and the monitor emits alarm tones if the stress level indicated by the monitored heart rate falls outside the levels for 60% and 80% of maximal oxygen uptake.

While the art relating to such devices is therefore a well-developed one a need still remains for a personal monitor and process capable of meeting the demands of an industrial environment.

Accordingly, it is an object of this invention to provide a personal monitor and process for work and heat stress which will measure physiological responses to the stresses and provide recommendations to the user in an industrial environment.

It is another object of the invention to provide such a personal monitor and process which will neither provide the user with a multitude of inappropriate warnings, nor allow the user to exceed safe limits without providing a warning to the user.

It is still another object of the invention to provide such a personal monitor and process which combines measurements of different body parameters to provide reliable indication of work and heat stress to the user.

This invention therefore relates to a device and process which helps a user avoid excessive heart rates and increases in body temperature during heat stress and heavy work exposures. More particularly, it relates to a device and process that measures physiological responses to work and heat stress and provides recommendations to the user in real time. Most especially, it relates to such a device and process in which the user is provided the recommendations to make an intelligent decision to control his or her exposure to the stress. It further relates to such an apparatus and process which is useful in an industrial environment.

The attainment of these and related objects may be achieved through use of the novel personal monitor, and process of this invention.

According to one aspect of the present invention a personal monitor of the kind defined hereinbefore at the beginning is characterised in that the information processing means under software control analyses the heart beat information incrementally over predetermined time intervals through moving time averages with a plurality of different time bases by incrementing the time base of the moving time averages. Preferably an insulated skin temperature sensor is provided to produce output electrical signals at least approximately indicating a user's body core temperature, said information processing means being connected to receive body core temperature information corresponding to the body core temperature output electrical signals, said information processing means further being configured to compare the body core temperature information against a stored body core temperature limit, and said information processing means further being configured to cause said means for providing the indication to the user to provide the indication when the user's body core temperature exceeds the stored body core temperature limit.

Also, in a preferred embodiment of the invention, the temperature sensor comprises a thermally and electrically conducting member having a bare, outwardly facing, unbroken surface configured and positioned to make direct contact with the skin of a person whose temperature is to be measured, a temperature sensitive solid state device thermally and electrically directly connected to an opposite surface of said thermally and electrically conducting member for producing an output electrical signal which is a function of temperature and a sufficient body of thermally insulating material surrounding a remainder of said thermally and electrically conducting member to provide the output electrical signal indicating insulated skin temperature as an approximation of the person's body core temperature, said thermally and electrically conducting member comprising both a thermal contact for the person's skin and a reference electrode of said temperature sensor.

EP 0 319 551 B1

According to another aspect of the invention there is provided a process for monitoring the heart rate of an individual which comprises measuring the individual's heart beats, analysing the heart beat information to obtain a physiological demand, comparing the obtained physiological demand against a predetermined physiological demand limit, and providing an indication to the individual when the predetermined physiological demand limit has been exceeded, characterised by analysing the heart beam information incrementally over predetermined time intervals through moving time averages with a plurality of different time bases by incrementing the time base of the moving time averages. A preferred process further comprises measuring the individual's insulated skin temperature as an approximation of body core temperature during the predetermined time intervals, comparing the measured approximation of body core temperature against a predetermined temperature limit and providing an indication to the individual when the measured approximation of body core temperature exceeds the predetermined temperature limit.

The invention will now be described by way of example with reference to the accompanying drawings, in which:-

Figure 1 is a perspective view of a first portion of a personal monitor in accordance with the invention.

Figure 2 is a perspective view of a second portion of the personal monitor, with a partial cutaway to show interior detail.

Figure 3 is a schematic diagram of a first electrical circuit used in the portion of the personal monitor shown in Figure 1.

Figure 4 is a schematic diagram of a second electrical circuit used in the portion of the personal monitor shown in Figure 1.

Figure 5 are waveform diagrams useful for understanding operation of the electrical circuit of Figure 4.

Figure 6 is a perspective view of a second portion of the personal monitor of Figures 1-4.

Figure 7 is a schematic diagram of a third electrical circuit used in the second portion of the personal monitor shown in Figure 6.

Figure 8 is a schematic diagram of a fourth electrical circuit used in the second portion of the personal monitor shown in Figure 6.

Figure 9 is a perspective view of a third portion of the personal monitor of Figures 1-4 and 6-8.

Figure 10 is a flow chart useful for understanding operation of software forming a part of the invention.

Figure 11 is a diagrammatic representation of certain information stored and used in operation of the invention.

Figure 12 is a diagrammatic representation of certain other information stored and used in operation of the invention.

DETAILED DESCRIPTION OF THE INVENTION

PHYSIOLOGICAL BASIS

This section describes the physiological basis on which the PM algorithm was based. Physiological strain is the body's response to a stress. The stress in heat stress is the combination of work demands, clothing requirements and the environment. In addition to sweating, the most apparent physiological strains in response to heat stress are body temperature and heart rate. Basically, the accumulation of heat is reflected in body temperature while the ability to perform work and dissipate heat is reflected in heart rate. While body core temperature and heart rate correlate with each other, they measure different physiological strains, and therefore add information about physiological state when both are considered. The Personal Monitor (PM) attempts to account for both body temperature and heart rate in order to present an alert for excessive physiological strain. Based on laboratory evaluation there is also evidence the PM is sensitive to the onset of symptoms which is also reason to terminate a heat stress exposure.

The algorithm uses insulated skin temperature and heart rate history to determine the alert state. These two physiological measures are treated independently and the alert state is determined by the measure that reaches the alert threshold first.

The insulated skin temperature (called disk temperature) is a physiological variable developed to predict overexposure to heat stress as a surrogate measure for body core temperature. There is a very high correlation between core temperature under two different conditions of heat stress.

The next step is to establish a safe limit for core temperature. The National Institute for Occupational Safety and Health (NIOSH) and the World Health Organization have recommended limiting heat exposure so that core temperature does not exceed 38.0 °C for daily, prolonged periods, i.e., for chronic work under heat stress conditions. WHO suggested that "in closely controlled conditions the deep body temperature may be allowed to rise to 39 °C (102 °F)". There is an increased risk for heat illness at the higher core

4

temperature as well as increasing incidence of unsafe behavior linked to heat stress. Heat stroke is associated with core temperatures above 40°C.

The action alert threshold for core temperature was selected to be 38.5°C, because the PM represents a means to closely control the heat stress exposure and the alert threshold allows time to terminate the exposure. First, this limit allows a greater exposure to heat stress above acceptable levels for chronic exposures. Second, it allows five to ten minutes to leave an area of heat stress.

With a core temperature limit of 38.5°C specified, a disk temperature threshold for the alert could be determined. To set an action alert threshold for disk temperature that protects most workers from exceeding a core temperature of 38.5°C, two decisions were made. First, analysis indicated that the relationship of core temperature to disk temperature is different for different clothing. For this reason, two classes of clothing were defined: (1) work clothes or single cotton coveralls, and (2) double cotton coveralls or impermeable clothing (plastics) over coveralls. Second, disk temperature is not an exact measure of core temperature, but a surrogate. This means that there is a certain amount of error associated with limiting core temperature by disk temperature. For each clothing class or ensemble, a disk temperature was selected so that for 95% of the cases core temperature was less than 38.5°C. The selected disk temperature was the action alert threshold. The action threshold for work clothes or cotton coveralls is 38.2°C, and for double cotton coveralls or impermeable coveralls over cotton coveralls, it is 38.5°C. These values are the action alert thresholds in the PM algorithm, which indicate a time to terminate the exposure.

The design objectives for the PM called for a warning alert to give preliminary notice that there has been an increase in physiological strain, and reset criteria that would allow the alert status to change from warning to acceptable. The warning alert should allow about 10 minutes warning prior to an action alert under typical conditions of heat gain. The warning thresholds were 38.0 and 38.1°C for work clothes/cotton coveralls and double cottons/impermeable clothing, respectively. If the strain is reduced after a warning alert, the PM will reset to the acceptable indicator. The reset thresholds were set to be 0.2°C less that the warning level. The 0.2°C drop indicates a significant drop in temperature and avoids problems with temperature sensor fluctuation or toggling around the threshold (e.g., changing between 37.9 and 38.0°C).

The purpose of the heart rate criteria is to relate the information gained from monitoring heart rate to the cardiovascular strain of heat stress exposure. This is accomplished by examining the relationship between work demand and endurance time, then relating endurance time and heart rate through work demand, and finally proposing heart rate criteria to mark extensive strain. In addition, recovery heart patterns can be used to judge the level of cardiovascular adjustment to heat and work stress.

As a person performs physically demanding work, the muscles demand oxygen, which is delivered by circulating blood. The increased demand for oxygen and, therefore blood flow, is achieved by increasing the heart rate. As work demands increase, oxygen demand increases to support muscle metabolism and heart rate rises to increase blood flow. If a person is subjected to increasing levels of work (and oxygen demands), his or her heart rate will reach a maximum value, which is also marked by a maximum utilization of oxygen (or maximum aerobic capacity, MAC).

A common observation is that the same task can be easily performed by one worker while another will have difficulty. The relative difficulty of performing a work task depends on how the oxygen demand of the task compares to the individual's MAC. For work demands and commensurate oxygen demands less than the MAC, the relative difficulty is related to the oxygen demand expressed as the percent of the maximum aerobic capacity (%MAC). A very fit worker (high MAC) will have less difficulty performing the same task than a less fit one because the former worker will be working at a lower fraction of his MAC.

If the work requires an oxygen demand that is 30% of the maximum, the task is considered light and can be done for hours, but if it is 75% of the MAC, the task can only be performed for about 20 minutes under a great deal of physical duress that will lead to exhaustion. Many laboratory studies have been performed to examine the relationship between time to exhaustion (endurance time) and %MAC. In the typical study, an individual is given a constant work task requiring a steady rate of oxygen consumption (e.g., treadmill walking or pedaling a bicycle-ergometer). The work is performed until exhaustion, and the time is noted. The endurance time plotted against % MAC gives similar curves for all individuals. Endurance time decreases exponentially with %MAC.

An average person can work about 20 minutes at 80% of his/her MAC before exhaustion. This increases to 40 minutes at 70% MAC and 80 minutes at 60% MAC. From the job design point of view, the job should not require more time than the endurance time. So a work task requiring 70% MAC of a person should not exceed 40 minutes. Alternatively, if a minimum work time is required, the actual %MAC must be lower than that %MAC associated with the endurance time that equals the work time. For instance, if 40 minutes are required to perform a task, the actual %MAC should be less than 70% MAC for any worker who is expected to complete the task.

There is a range of endurance times (ET) for any particular value of %MAC. In order to be protective of most of the population, a conservative line can be drawn to include all of the ET data for a given %MAC, which follows the equation

$$\log_{10}(ET) = 4.0 - 4.0(\%MAC/100\%) \qquad (1a)$$

$$-\%MAC = 25\%[\log_{10}(ET) - 4.0] \qquad (1b)$$

where ET is endurance time in minutes and the term (%MAC/100%) is the ratio from 0 to 1 that the oxygen demand is to the MAC. Equations 1a and 1b are the same relationship expressed in two ways. The meaning of the relationship can be interpreted as follows. For any given %MAC, most workers would be able to perform at that relative level of work for at least a time of ET from Equation 1a. Or, if a work demand required a time of ET, it can be done by anyone who is working at or below the respective value of %MAC from Equation 1b. So a worker can work at least 10 minutes at 75% MAC; or a job requiring 10 minutes of work can be performed by anyone working at or below 75% MAC.

As a practical note, a pattern of different work demands with different oxygen demands can be averaged together over a time interval, and the average %MAC computed. The average %MAC is used to estimate the expected endurance time, and this endurance time is compared to the actual time requirement (the time base for the average). For instance, 5 minutes at 80% MAC can be averaged with 20 minutes at 50% MAC for an average demand of 56% MAC over 25 minutes. This average rate can be sustained because it is less than the endurance time of 100 minutes associated with 56% MAC. This averaging technique cannot be used if the work demand in a shorter period would mean the endurance time is exceeded. For instance, 30 minutes at 80 %MAC cannot be averaged with 30 minutes at 40% MAC for an average of 60% MAC over 60 minutes. The individual would reach exhaustion during the 80% MAC segment, because the endurance time for 80% MAC is twenty minutes.

Based on this averaging principle, an incremental analysis can be performed to assess whether a job may cause exhaustion. In the incremental analysis, the relative oxygen demands (%MAC) over small intervals (i.e., 5 minutes) are examined to determine if the associated endurance time is less than the time interval. That is, for any 5 minute interval, the average %MAC should not exceed 83 for the individual as calculated from Equation 1b, using 5 minutes for ET. The time step is then increased (e.g., 10 minutes) and the average %MAC for that interval is calculated. Once again, if the average %MAC is less than 75% MAC (value from Equation 1b for ET = 10 min), there should not be any excessive physiological demand (exhaustion). By incrementing the time base of the average out to the working time itself, all possible combinations of work can be examined in terms of their effect on exhaustion. This means that short periods of high demand are considered as well as long periods of lower average demands.

Because of the exponential nature of the relationship, the time intervals can be increased geometrically to reduce the number of averaging intervals without losing protective ability. So starting at 5 minutes, which is the smallest practical interval, the intervals can follow 10, 20, 40, 80,... minutes. As an approximation of the geometric progression, 7 averaging intervals were selected for the PM algorithm: 5, 10, 20, 30, 45, 60, and 90 minutes. From Equation 1b, the highest %MAC that can be sustained for these intervals is 83, 75, 67, 63, 59, 56, and 51 %MAC, respectively. For work scenarios where the PM would be used, there is no need to extend the averaging intervals beyond 90 minutes because few exposures will go to 3 hours (180 minutes) of steady work.

The next step to developing an algorithm for heart rate is to relate heart rate to %MAC. This step is required because it is not feasible to know the MAC of each potential user of the personal monitor as well as the oxygen demands for all possible tasks to be performed so that %MAC can be known.

As a person moves from a resting oxygen demand to MAC, heart rate increases from the resting rate ($HR_{rest}$) to the maximum heart rate ($HR_{max}$). The average resting heart rate is 75 beats per minute, and does not change much for age, race or sex. For the general population, $HR_{max}$ decreases with age, and is reasonably well predicted by:

$$HR_{max} = 220 - age(years) \qquad (2)$$

An implication of equation (2) for the population at large is that the average $HR_{max}$ for a given age is 1 beat per minute (bpm) less than those a year younger. This also means average MAC decreases because of a lower ability to deliver blood and oxygen to the muscles, as is the case.

Heart rate reserve (HRR) is the difference between $HR_{rest}$ and $HR_{max}$. Any heart rate (HR) can then be expressed as percent heart rate reserve %HRR by:

$$\%HRR = [(HR_{max} - HR)/(HR_{max} - HR_{rest})]x100\% \qquad (3a)$$

or

$$\%HRR = [(HR_{max} - HR)/HRR] \times 100\% \qquad (3b)$$

$\%HRR$ is zero at rest and 100 at $HR_{max}$.

There are three factors to consider: (1) MAC(100%MAC) occurs at $HR_{max}$, (2) the oxygen demand is about 10%MAC, and (3) heart rate increases linearly with %MAC. These three facts suggest a basic approximation used in the PM: %HRR is equal to %MAC. If someone is using 50% of his MAC to do work, he will also use 50% of his HRR. To illustrate these relationships, suppose a 45 year old worker performs a task with a heart rate of 125 beats per minute (bpm). First, his $HR_{max}$ = 175 (=220 - 45, Equation 2) and he has a resting rate of 75. He has an HRR of 100 bpm (175 - 75 = 100). If his heart rate is 125 bpm, he is using 50% of his HRR([(175-125)/100] x 100%, Equation 3b). This means he can work for at least 100 minutes (Equation 1a). Knowing HR then allows the endurance time to be estimated through %HRR and %MAC. This demonstrates that it is not necessary to know the oxygen demand of the task or the individual's MAC to be able to estimate endurance time - only heart rate for the task.

The relationships can be used in reverse as well. If a minimum work time is known and equated to ET, the highest expected work demand expressed as %MAC can be estimated from Equation 1b, and therefore the %HRR. With the %HRR and assumptions about $HR_{rest}$(=75) and $HR_{max}$ (Equation 2), a limit on heart rate can be suggested. As an example, suppose the work time is 20 minutes and the worker is 45 years old (as above). The highest %MAC is 67 for ET = 20 from Equation 1b. This then means that the average heart rate for the 20 minutes should not exceed 67% HRR. Equation 4 is a rearranged version of Equation 1b.

$$HR = HR_{rest} + HRR(\%HRR/100\%) \qquad (4)$$

Sixty-seven %MAC translates to a heart rate of 142(=75 + 100[67/100]), Equation 4.

The preceding discussion, however, has not addressed heat stress. It is known that the added burden of heat stress on the cardiovascular system is also reflected in heart rate, and that heart rate increases due to heat stress can be treated as an increment in %MAC. In other words, the physiological strain reflected in heart rate is the same whether the heart rate is due to work alone or a lesser level of work in combination with heat stress. Therefore, the effect of work and heat stress on heart rate are additive, and the overall effect on heart rate can be used to estimate a %MAC through %HRR. The %MAC can then be used to predict a maximum work time or endurance time.

Two assumptions come into play, therefore, in using heart rate to monitor physiological strain due to work under heat stress. First, the effects of heat stress on heart rate are additive to the heart due to work alone. Second, heart rate can be used to reflect the equivalent %MAC and thus the endurance time is short for high %MAC and increases with lower values of %MAC. The resulting principle is that high heart rates (high demands, reflected as the combination of metabolism and heat stress) can be safely sustained for short periods of time and lower heart rates (lower demands) for longer periods.

For the PM, a heart rate threshold is a level above which an average heart rate for a specified period of time triggers an alert. For a short time, the PM should tolerate a high heart rate due to heavy work and/or high heat stress. This can be achieved by using a short averaging period with a high threshold. In contrast, the PM must be sensitive to a moderate demand (lower HR) over a longer period. Therefore, a longer averaging period can be used with a lower threshold.

Averaging avoids problems commonly found with single, static thresholds used in currently available heart rate monitors. If a static criterion is set high enough to allow bursts of high demand, then it will miss longer terms of moderate strain. If it is set low for moderate strain, it will alarm for brief, but allowable, levels of high demand. The PM accounts for the continuum of strains between these two situations by employing multiple averaging periods. Thresholds can be selected for each of the multiple averaging periods for heart rate.

Multiple averaging periods and thresholds are calculated and updated through the technique of moving-time-averages (MTAs) of heart rate. The application of MTAs represents a novel approach for heart rate monitoring. An MTA-5 (moving-time-average over 5 minutes) represents the average heart rate for the past five minutes, and can be used to examine a burst of high demand. If a heart rate is high for a couple of minutes, the MTA-5 will quickly approach the high rate. When it reaches a critical value, it will cause an alert. But if the burst of high demand is too short to cause a concern, the MTA-5 will not reach the critical level. In this way, it can be sensitive to high demands without overreacting to brief transients. At the other extreme is an MTA-90 (90 minute moving-time-average). Even sustained levels of high heart rate are hidden, but gradual increases due to long term, moderate demands that will cause fatigue are accounted for. A threshold or critical value for MTA-90 can be much lower, without causing an alert until the long-term demand is judged excessive.

The PM uses 7 MTAs on time bases of 5, 10, 20, 30, 45, 60 and 90 minutes to provide a steady progression of protection from short term, high demands to long term, moderate demands. The MTA intervals were taken as an approximation of a geometric progression (successive doubling) from 5 minutes.

The effects of moving time averages on smoothing out peaks and identifying trends can be seen in the following example. Consider an operations supervisor performing an inspection of the containment building of a nuclear power plant immediately following a hot shutdown. This requires much ladder and stair climbing (high heart rates) as well as relative inactivity. MTA-5 follows the short-term trends of HR very well without being unduly influenced by very short peaks. MTA-90, on the other hand, follows the steadily increasing cardiovascular load caused by the long-term effects.

The action alert thresholds for the MTAs were selected using two factors: (1) age for adjustments in $HR_{max}$ and (2) %MAC for an endurance time equal to the time base of the averaging period. Three age groups were judged to be sufficient to reflect the effects of age on $HR_{max}$. Two groups would not account for effects of age, and the variation in $HR_{max}$ for a given age defeats any advantage of more groups. HRRs were then estimated for each group assuming a typical resting rate of 75 bpm and an $HR_{max}$ for the midrange age. The groups with respective HRRs and midrange age are:

*Young:     <35 years with HRR = 118(27years)
*Middle:    36-50 years with HRR = 102(43 years)
*Older:     >51 years with HRR = 87(58 years)

For each MTA time base, ET was set to the time base and the %MAC (and hence the %HRR) was calculated from Equation 1b. For the 7 averaging intervals - 5, 10, 20, 30, 45, 60, and 90 minutes, the highest %MACs that can be sustained for these intervals are 83, 75, 67, 63, 59, 56, and 51 %MAC, respectively. For each age group, the heart rate for each %HRR, which equals %MAC, was calculated using Equation 4. This was taken as the action alert threshold. The heart rate thresholds for the older age group were adjusted downward by about 2 bpm based on the laboratory tests and other data that suggest a greater loss of ability to cope with heat stress than would be expected from an age related decrement in cardiovascular capacity.

The warning alert thresholds were selected to give about 10 minute warning to the action alert threshold if the heart rate was maintained at moderate demands. The reset levels were selected to indicate a sustained drop in heart rate from the warning levels. They appear to be adequate from the laboratory tests.

Heart rate recovery patterns were introduced by Brouha at duPont as a method to evaluate the physiological strain experienced by an individual performing physically demanding work. They also apply to conditions of heat stress. The criteria were revised by Fuller and Smith. The recovery heart rate method requires a worker to stop working and sit down for 3 minutes. Three 30 second pulse counts($P_n$) are taken during the second half of each successive minute as follows:

*$P_1$:     30 to 60 seconds after sitting
*$P_2$:     90 to 120 seconds after sitting
*$P_3$:     150 to 180 seconds after sitting

Each of the three pulse counts are doubled to give a pulse rate. Fuller and Smith found that a $P_3$ rate less than 90 bpm indicated full recovery and that the preceding work did not have an excessive demand; that is, it was acceptable.

If $P_3$ was greater than 90, the worker was accumulating a physiological debt with the implication that he cannot continue indefinitely. Looking further, if the drop from $P_1$ to $P_3$ is greater than 10 bpm, the recovery is marginal. This means he can continue to work, but exhaustion may occur later. This is analogous to the warning alert. If the drop is less than 10 bpm, there is no recovery and work should cease, equivalent to an action alert.

Based on the laboratory data for recovery patterns near the termination point, the above procedure was further modified. A "no recovery" category is assigned by the PM algorithm if $P_3$ is greater than 120, regardless of the difference from $P_1$.

HARDWARE

The personal monitor (PM) is comprised of three distinct components: sensor module, monitor module and alert module. Each of these will be described in detail.

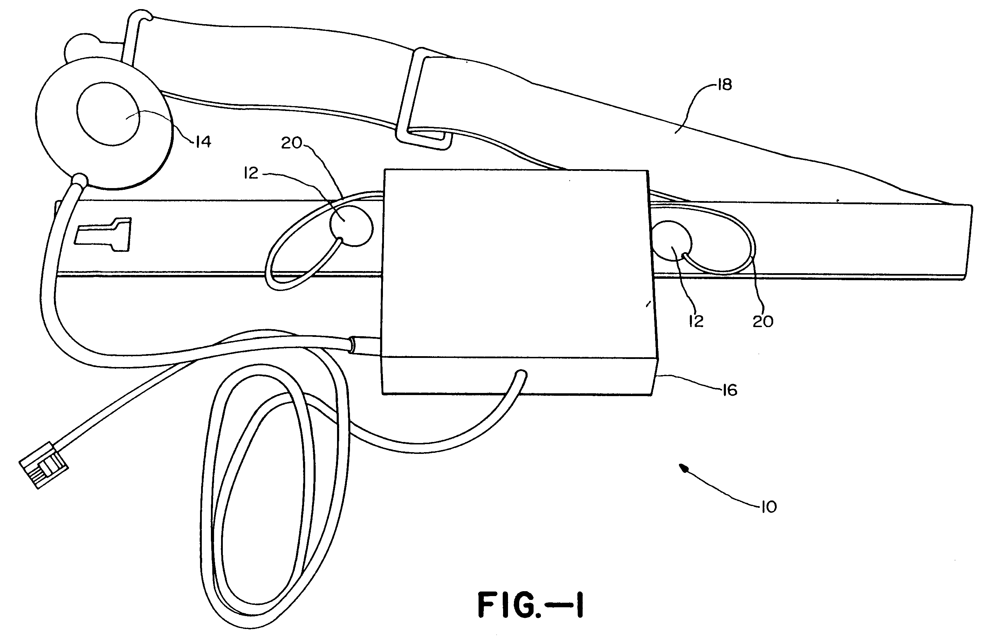

The sensor module 10 is shown in Figure 1. The sensor module 10 contains EKG sensors (electrodes) 12, temperature disk (insulated skin temperature) 14, and an electronics package 16 for the sensors 12 and 14. The sensor package 16 is mounted on a harness 18 worn on the chest. Placed on the inside of the harness 18 are the EKG electrodes 12 and the temperature disk 14, all of which must be in contact with the skin.

The primary goal in the selection of the EKG electrodes 12 was to select or design ones that could be used to obtain a good EKG signal with a minimum amount of skin preparation. Good readings can be obtained if the impedance between the two electrodes 12 is lower than 10K ohms.

The electrodes 12 selected for the personal monitor are commercially available as an integral part of a chest strap harness 18. This strap 18 is part of an exercise heart rate monitor developed by AMF and marketed by Computer Instruments Corporation. It uses two flexible conductive materials segments about 3 cm x 6 cm on the inside of the strap 18 for the electrodes 12. A button snap through the material and the harness 18 establishes an electrical connection to the outside. The PM sensor module 10 uses leads to the button snaps for the EKG electrodes 12.

If the electrodes 12 are simply placed on the chest over dry skin, the impedance is too high (>10K ohms) to obtain a good EKG. After a couple of minutes, enough sweat has accumulated under the electrodes 12 to reduce the impedance to acceptable levels. To obtain an acceptable EKG signal immediately, a small amount of conducting lotion or water can be spread on the electrodes 12 before donning them. No skin preparation (e.g., rubbing, cutting away hair, etc.) is necessary.

Alternative electrodes that can be used in place of the electrodes 12 are the pregelled, disposable type frequently used in hospitals and for exercise stress tests. The basic concept is a soft foam disk with adhesive surface that is used to attach the electrode to the person. The center of the disk has a conductive jelly to form an electrical contact with the skin. A third type of electrode that could be used is a conductive gelatin type used with an exercise heart rate monitor by Biotechnology, Inc.

Three electrodes (active, reference and ground) are needed to measure the EKG. The active electrode 12 is placed over the heart (left side of the chest). The reference electrode 12 is placed on the right side of the chest. The temperature disk 14 also serves as the ground electrode, and it is placed outside of the line formed by the reference and active electrodes 12. In this fashion, the copper disk is both a thermal and electrical conductor.

The harness 18 is easily donned and doffed by one person. Generally, it is then slid into correct position on the chest and the temperature sensor 14 is inserted under the harness 18 on the right side. The harness 18 can be hand washed to sanitize it.

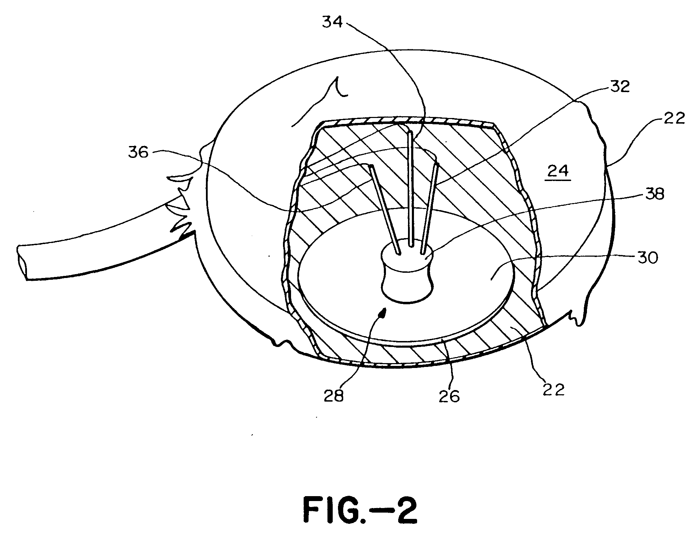

The temperature sensor 14 is shown in Figure 2. A styrofoam disk 22 which is 0.8 cm thick with a 4.2cm diameter is the insulating body of the sensor. On the surface 24 facing the skin there is a 2.5 cm diameter copper disk 26. A temperature sensitive solid state device 28 (National Semiconductor LM34CA) is soldered to the internal side 30 on the copper disk 26 and surrounded on the other sides by the styrofoam disk 22. The solid state temperature sensor 28 has a low output impedance and a linear voltage output proportional to temperature (T) as expressed by:

voltage (mV) = 10 x (temperature in °F).

For example, the output is 0.950 V at 35°C (95°F) and is 1.040 V at 40°C (104°F), for a change of 90 mV over the range of typical disk temperatures. If desired, a thermistor could also be used, but the solid state device 28 has a comparable temperature sensitivity, is more rugged, and has a lower power requirement.

There are three electrical leads 32, 34 and 36 from the temperature sensor 28. The first lead 32 is the electrical common (ground) lead that is connected to the metal case 38 of the sensor. This lead 32 is also used as an electrical conductor in its role as ground electrode. The second lead 34 is for the supply voltage (+5 volts). The third lead 36 is the voltage output, which is proportional to temperature.

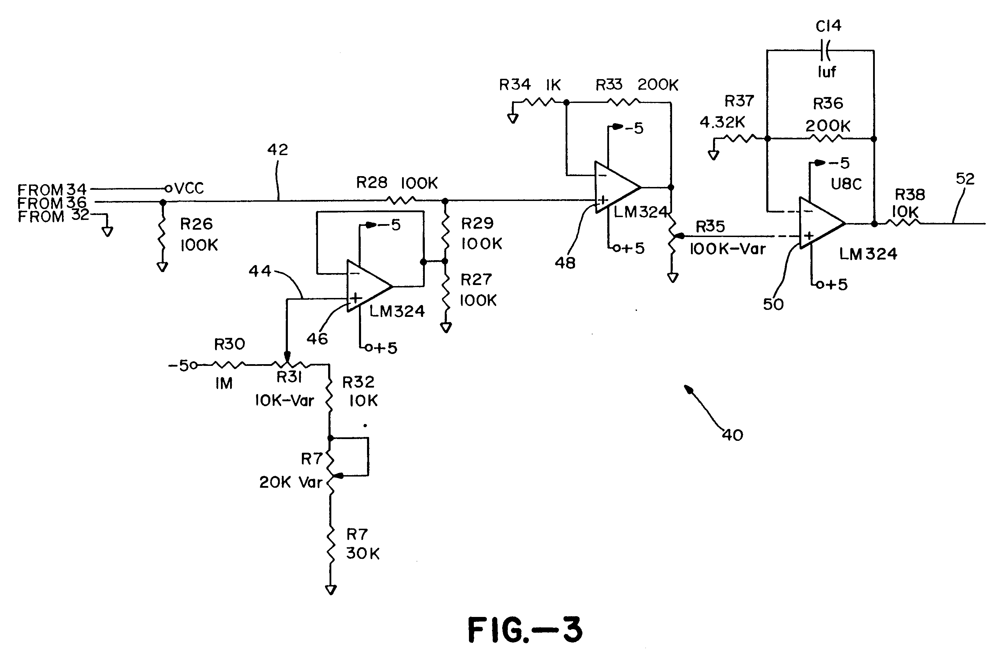

Sensing circuit 40 is shown in the diagram of Figure 3. The first step is to sum the voltage output from the device 28 on line 42 with a bias voltage on line 44 in summing amplifier 46 so that a zero adjustment is available. Using the adjustable resistors R31 and R7, a nominal bias voltage of -975 mV is summed with the temperature device output voltage so that the voltage entering amplifier 48 is zero when the device is at 36.4°C (97.5°F).

9

Another variable resistor R36 is used to adjust the gain of amplifier 50. The amplifier 50 gain is set so that a one bit change in an analog-to-digital (A/D) converter in the monitor module is equivalent to 0.025°C. The monitor module has an 8-bit A/D converter over a range of 0 to +5 volts, which means that it gives an numerical value for 0 for 0 volts and 255 for +5 volts. That is, there are 51 output units per volt input to the A/D converter. Using the target sensitivity of 0.025°C/unit, an increase of 3°C over 36.4°C (or 39.4°C) should have an A/D reading of 120 (=3/0.025). The gain is adjusted so that a temperature of 39.4°C on the device 28 will yield 2.353 volts (5x120/255) at the amplifier 50 output 52. The amplifier output is the input to the A/D converter. The device 28 is calibrated with a water bath over the important range of temperatures (37° to 39°C), and the calibration curve is placed in nonvolatile memory (EEPROM) as the slope and intercept of a least squares fit.

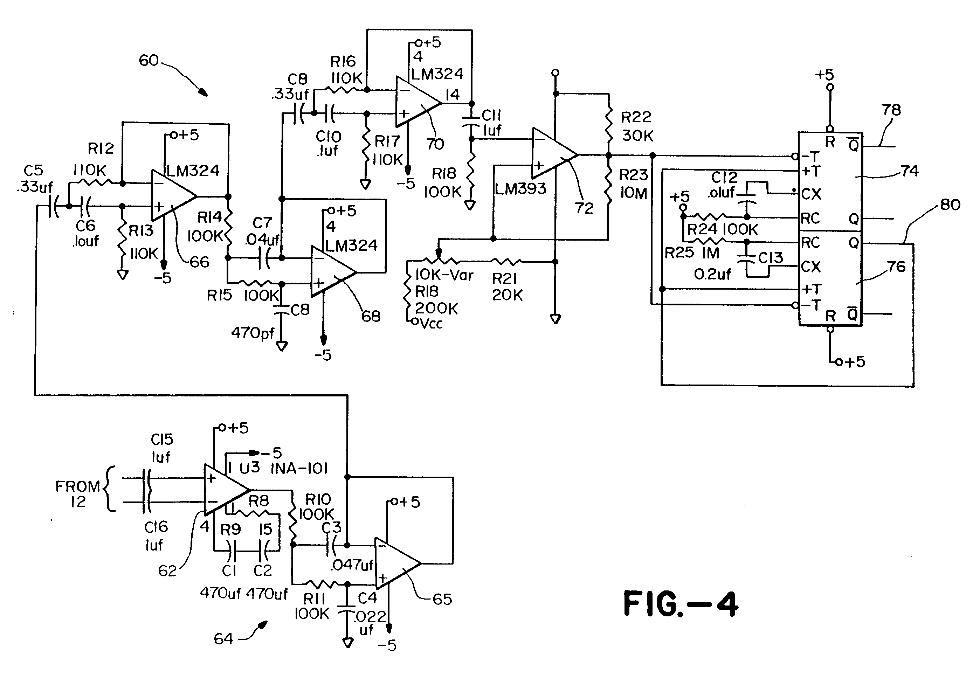

The role of the sensor module 10 in the determination of heart rate is to detect the occurrence of a heart beat and send a signal the the monitor module when one occurs. Heart rate detection circuit 60 in Figure 4 relies on being able to detect the occurrence of a QRS complex, which is a very distinctive part of the EKG. It is a rapidly changing wave with a high amplitude (usually between 2 and 5 times larger than the other, more slowly changing waves).

As mentioned above, there are three electrodes involved. One is the ground electrode 14, which is also the temperature disk. It is connected to the instrument ground and provides stability to the signal. The other two electrodes are the positive (active lead) and negative (reference lead) inputs from EKG sensors 12 to an instrumentation grade differential amplifier 62 (Burr-Brown) as shown in Figure 4. The signal is amplified by a factor of 1000 (from millivolts to volts) and is also passed through a relatively broad bandpass filter 64. The signal then passes through four active filters 65, 66, 68 and 70 that together provide a narrow bandpass filter designed to allow the moderate frequency (nominally 100Hz) QRS wave to go through, while blocking lower frequency EKG waves, electrical noise, such as 60 Hz electrical noise in the environment, baseline drift due to changing electrode impedance and any higher frequency noise, such as 400Hz instrumentation noise. The filter cutoff frequencies were designed empirically. The cutoff frequencies for the bandpass filter were adjusted by looking at the effect on the QRS wave. The frequency of the two high pass filters was increased until the amplitude of the QRS was affected; and in a similar manner, the frequency of the two low pass filters was lowered. The filtered EKG/QRS signal is then sent to a high gain comparator amplifier 72 that has a zero output until the input reaches a predetermined level. If the signal goes above that level, the output goes very quickly to +5 volts. Thus, when the QRS signal reaches the threshold, the amplifier 72 sends out a positive square wave to trigger a pair of monostable multivibrators 74 and 76.

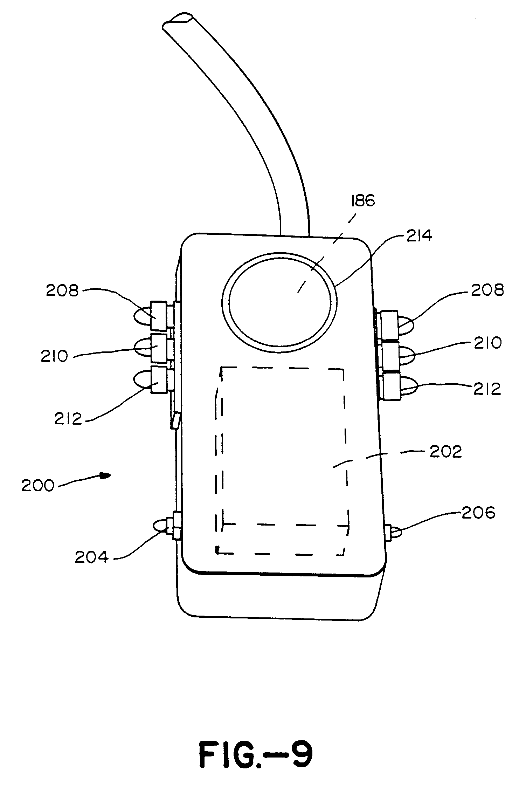

The mulitivibrator circuit 74 provides a steady high signal (+5 volts) until the input trigger receives a positive pulse. The positive pulse triggers the beginning of a zero volts square wave from multivibrator 74 output 78. The output 80 of the other multivibrator 76 stays high for about 250 msec, which prevents triggering the first multivibrator 74. The relatively long high window of the second multivibrator 76 prevents a triggering of the first multivibrator 74 at frequencies above 255 times per minute, which is higher than any heart rates that will be observed. In addition to advancing a counter (see monitor module electronics), the low pulse from the first multivibrator output 78 allows LEDs 208-212 (Figure 9) to blink with each heart beat (see alert module electronics). The width of the pulse to the LEDs was selected so that there is a discernible flash on the LEDs.

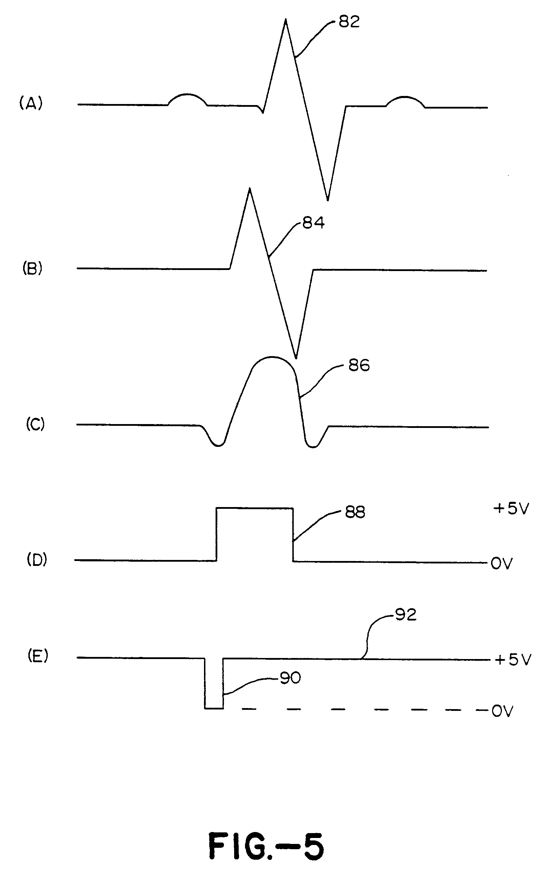

Figure 5 illustrates the effects of the detection circuitry 60 on the EKG signal. At the top, the raw EKG signal 82 for one heart beat is shown. After passing through the first, broad bandpass filter 64, the signal has a sharp, bipolar character, illustrated at 84 in (b). The four stage, narrow bandpass filter 65-70 removes the negative component and the slow waves, and broadens the positive wave, as shown at 86 in (c). The signal then goes to the comparator 72 from which the filtered signal creates a square wave 88, as shown in (d). This highly conditioned signal then triggers the multivibrator circuit 74 to send a clean zero volts square wave 90, shown in (e), on a normal +5 volts signal 92. The sensor module 10 (Figure 1) is supplied directly from the battery source. There is a +5 volts voltage regulator in the sensor module 10 that supplies the module integrated circuits.

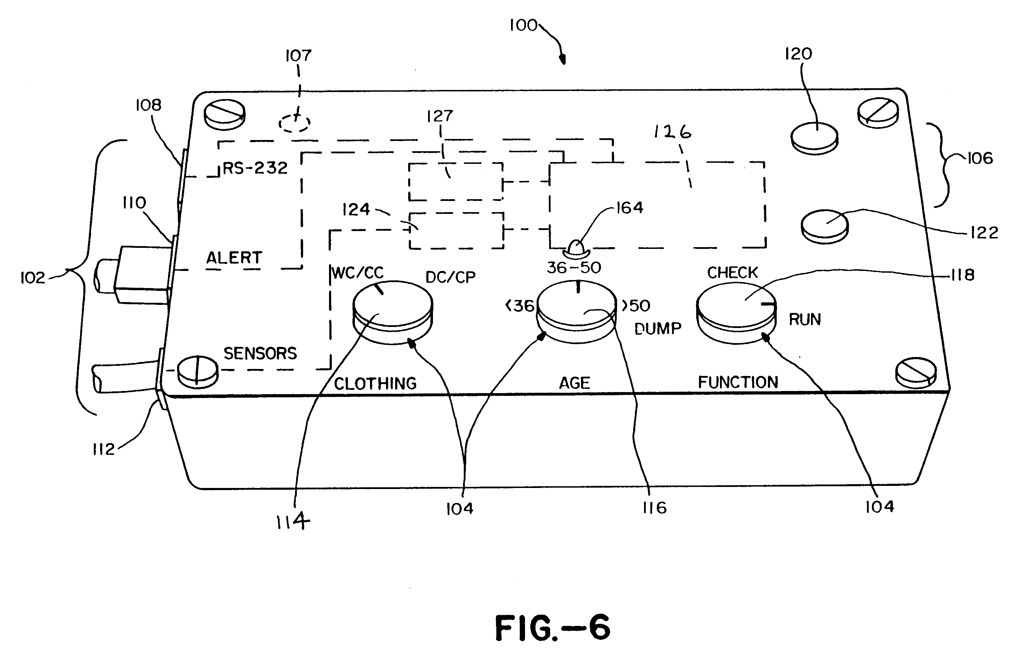

Monitor module 100 (Figure 6) has four component features: module connection ports and control switches, heart beat counter, microprocessor board and alert module electronics. The monitor module 100 has three connection ports 102 and five switches, 3 external switches 104 and 2 internal switches 106. The three modular phone jacks 102 are used for connecting the sensor and alert modules 10 and 200 to the monitor module 100 and for providing a communications port. In addition, there is an auxiliary battery connection port 107.

Communication port 108 is the top plug in Figure 6, which is labeled RS232. It is an RS232C port requiring a six wire phone plug attached to the special communications cable supplied with an Onset Computer datalogger. This connection is necessary for communication with the microprocessor for (1) monitoring PM performance during laboratory tests and demonstrations, (2) calibration check of the PM, (3) obtaining PM data using the DUMP function, and (4) communicating with the microprocessor for program check-out or modification.

Alert module port 110 is the middle port, labeled Alert. It is an eight connector jack to attach the alert module.

Sensor module port 112 is the bottom phone jack. It has four leads that attach to the sensor module 10.

Auxiliary battery port 107 is located on the top side of the monitor module 100. It allows for the connection of a second or alternative battery to power the monitor module. It can also be used to extend the service time of the PM.

The external switches 104 are located on the front surface of the monitor module. Clothing switch 114 is a two-position switch to select and indicate clothing ensemble: (1) work clothes or single cotton coveralls and (2) double cotton coveralls or impermeable (vapor barrier) clothing. Age switch 116 is a three-position switch used to indicate which set of MTA thresholds should be used. The three age groups are: (1) less than 36 years; (2) 36-50 years; and (3) greater than 50 years. Function switch 118 indicates the mode of operation for the PM. The details on mode are discussed in the section on Software below. The modes are: (1) DUMP mode; (2) CHECK which allows for a system checkout and calibration; and (3) RUN mode.

There are two internal switches 106, which can be accessed through taps in the front cover. DEMO/DUMP format switch 120 is an internal three-position switch located in the upper right corner of the module as shown. It serves two purposes. In the CCW position, all of the data stored during the use of the PM is sent to the communications port 108 during the DUMP routine (see Software). In the middle position, the MTA data are deleted from the dump output. In the CW position, two actions are possible. In DUMP, the MTA data are deleted from the dump output. In the RUN mode, program control passes over to an alert demonstration routine. This switch can be changed through the upper tap in the cover.

Baud rate switch 122 is located near the right side of the module 100 and in the middle as shown. It selects the communications port speed for data transmission: (1) 300 baud, (2) 1200 baud and (3) 9600 baud. If the terminal (printer, computer, etc.) does not support the XON/XOFF communications protocol, it is best to communicate at 300 baud. At the higher baud rates, output data (especially during the DUMP routine) may become garbled or lost. Communications specifications include 8 bit words with one stop bit and no parity check. This switch can be changed through the lower tap in the front cover. The baud rate is changed to the new value at powerup or after an entry into the DUMP mode (see Software).

Heart beat counter 124 is a typical integrated circuit with a trigger (called a clock) input, a reset control, and 12 output bits. The counter 124 will count each heart beat as sent from the sensor module 10 into the trigger input. This counter is reset to zero when a positive pulse is sent to the reset input. Only the first seven bits of the counter are used as input to microprocessor board 126. This means that values from 0 to 127 can be read by the counter 124. If the counter tries to go to 128 before a reset, it will roll over to zero and begin again. The counter 124 is read and reset every 30 seconds, which means that a heart rate up to 254 beats per minute can be counted, which is higher than is physiologically possible. The counter 124 is reset by the microprocessor 126 under the program control.

The microprocessor board 126 is a commercially available Tattletale Model II datalogger designed for OEM applications by Onset Computer. It uses a customized version of BASIC (TTBASIC, Version 1.72). The manual for this board describes the hardware and programming language. The important features of the microprocessor hardware are summarized below.

The datalogger hardware is contained on a 7.4cm by 12.7cm (3x5in.) printed circuit board. The hardware includes an 8 bit microprocessor 126 with a 32K EEPROM (electrically erasable programmable read only memory), 256K of dynamic RAM 127, 8 channel 8 bit analog-to-digital (A/D) converter, and a 14 pin digital I/O (14 bits of digital input and/or output). In addition there is an RS232C serial communications port. There are 21 locations to store numbers in nonvolatile memory (EEPROM) accessible from TTBASIC. When the processor is powered up (turned on), it loads the program stored in EEPROM into RAM 127 and begins execution of the program. Under the PM application, the ROM contains the TTBASIC interpreter and the PM software written in TTBASIC. The PM software and the TTBASIC reserved variables actually occupy about 60K of RAM, leaving the balance for storage of PM data collected during operation. During each minute of operation, the PM stores about 22 bytes of data or about 1.2K per hour. While the data storage was designed for laboratory and field trial tests, it may have applications for routine evaluation of individual stress and job demands in a broader heat stress management program.

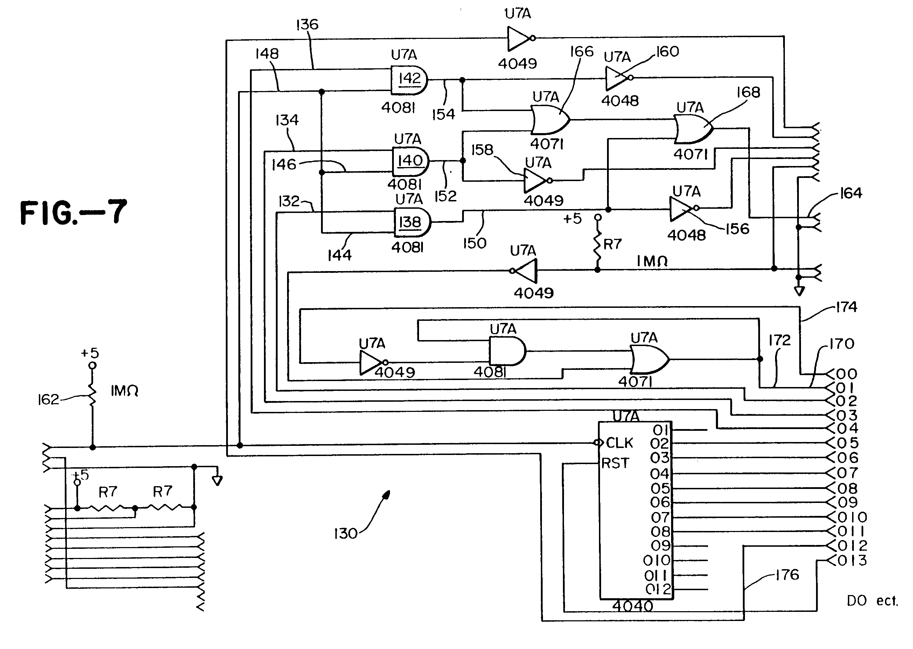

Figure 7 shows a circuit diagram for the alert module electronics 130, which are contained in the monitor module 100. The digital lines 132, 134 and 136 for the green, yellow and red LEDs are brought out to separate AND gates 138, 140 and 142. On the second legs 144, 146 and 148 of each AND gate 138-142 is the heart beat signal from the sensor module 10. The AND gate 138-142 outputs 150, 152 and 154 then go through a separate amplifier 156, 158 or 160 to drive their respective LEDs. When the program calls for an LED to be on, it will be on and flash off with each heart beat. (Remember that the heart beat line is at +5 volts except when a QRS wave is detected.) It should be noted that if the sensor module 10 is not attached, a pullup resistor 162 will keep the heart beat signal high. The circuit provides for a monitor module LED at 164 that also flashes with heart beat when any one or more of the alert module LEDs is turned on The outputs 150-154 are connected to the monitor LED output 164 through OR gates 166 and 168 as shown.

When command button line 170 is activated, logic line 172 supplying channel 1 of the digital I/O will be taken high (+5 volts). This line will remain high until a reset high signal (+5 volts) is sent out to line 174 on channel 0 of the digital I/O by the PM program.

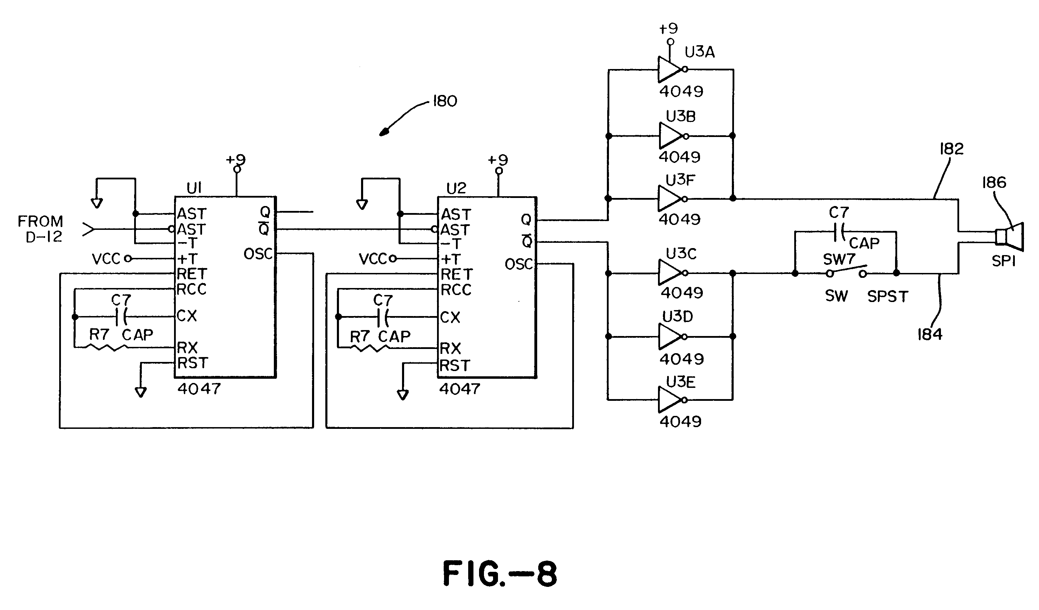

When an aural alarm is requested by the PM program, digital I/O channel 12 on line 176 will go to zero volts briefly which triggers a timer 180 (Figure 8). The timer 180 allows a tone to sound for 3 seconds. The sound is generated by alternating plus and minus legs 182 and 184 of a battery on the piezoelectric drive of a diaphragm 186 of the alert module (see also Figure 9).

The monitor module (Figure 6) has the LED 164 provided on the front of the module. This LED 164 is activated at the same time one or more of the LEDs 208-212 (Figure 9) on the alert module 200 is called. The primary purpose is to verify a good heart beat tracking without looking at the alert module. There may be some applications when the PM is used to gather physiological data and the alert module is not connected. When a battery is connected directly through the auxiliary battery jack 107 of the monitor module 100, the alert module 200 is not needed to power the PM (see alert module in the following section). The auxiliary jack 107 can also be used to prolong the PM power supply or to preserve data collected after the alert module is disconnected. (All information from a PM session is lost if power to the microprocessor 126 is interrupted.)

Alert module 200 is shown in Figure 9. It has four components: battery, command buttons, alert LEDs and tone generator. The alert module 200 carries a 9-volt transistor battery 202, which is the principal power supply for the PM. Battery voltage is checked by the software. Two buttons 204 and 206 are placed on opposite sides of the alert module 200. If both buttons are pressed simultaneously, the command logic signal on line 174 (Figure 7) is set High (+5). The PM program makes use of this signal to take special actions determined by the function switch 118 (Figure 6) position. Two sets of three alert LEDs 208, 210 and 212 are available, one set on each side of the module 200. From top to bottom, they are red, yellow and green. Which pairs flash with heart beats is under program control through the monitor module 100. A piezoelectric driven diaphragm 186 is inside the module 200, with an opening 214 to the outside. This provides the actual aural alarm at the command of the microprocessor 126 and is driven by electronics in the monitor module 100.

## SOFTWARE

The personal monitor uses an algorithm to assist an individual in determining exposure to heat stress. The algorithm is incorporated into a program to control data collection of disk 14 (Figure 1) temperature and heart rate, manipulate the data as necessary to obtain relevant information, and activate appropriate alerts. The program reflects the implementation of the algorithm within the prototype hardware framework. The following description of the PM software describes the critical measures and the decision-making structure.

The PM hardware described above is responsible for monitoring insulated skin (disk) temperature and heart beats (stored in a counter) and for providing the alert status and user control. The program, stored in an EEPROM (electrically erasable programmable read only memory) controls the datalogger microprocessor 126 (Figure 6).

The following list is the description of the critical variables for the PM as they are used in the algorithm.

$T_d$      Skin temperature taken at one minute intervals

HR      Heart beats counted over the 30 seconds, multiplied by 2 for rate

MTAn      Moving-time-average (MTA) HRs over past n-minutes (n = 5, 10, 20, 30, 45, 60, 90) (initially set to 75 and updated by HR every minute)

Green      Alert that indicates Acceptable (normal) physiological stress

Yellow      Alert that indicates a Warning level

Red      Alert that indicates an Action level

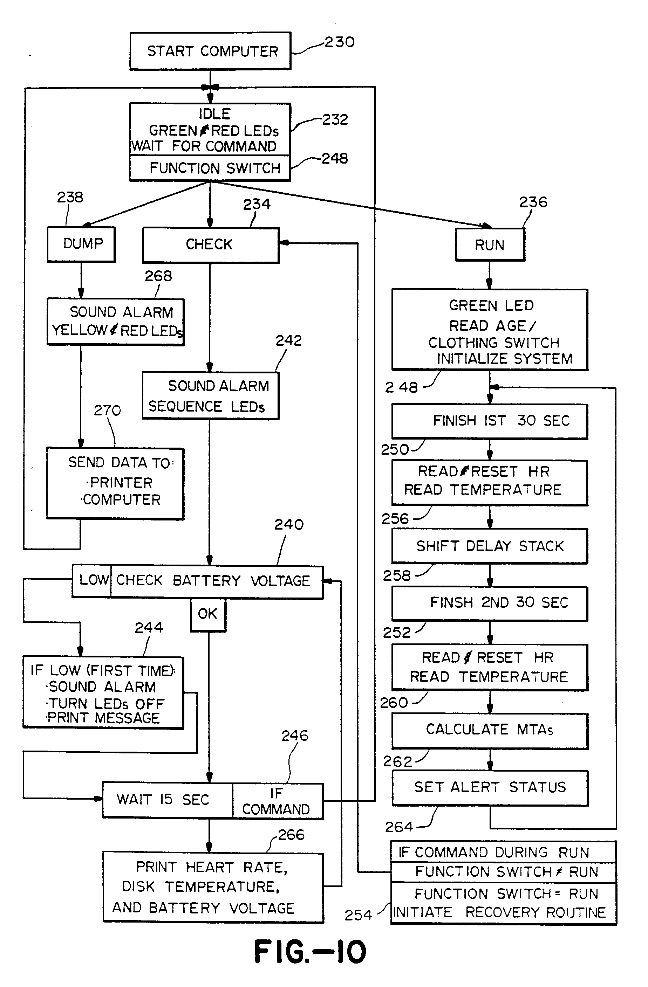

An overview of the program control process is shown in Figure 10.

## PM MODES AND ROUTINES

When the alert module 200 (Figure 9) is plugged into the monitor module 100 (Figure 6) (or when an auxiliary battery is attached), the microprocessor 126 is activated at 230. Its first step 232 is to load special factors from nonvolatile memory and turn on Green and Red LEDs 212 and 208. The LEDS will flash with heart beats if the system is receiving an EKG signal. The monitor (or computer) is in the IDLE mode waiting for the command buttons 204 and 206. After reading the function switch 118, the PM enters one of three modes 234, 236 or 238:

CHECK, which also allows a calibration check

RUN, which starts the PM to monitor physiological state and to control the alerts

DUMP, which allows the time course of the user's physiological state and the PM's alert information to be downloaded to a printer or another computer for inspection

Line numbers of the program code are given in the following discussion to link the discussion to the program. If the communications port 108 is used to connect the PM to a terminal, PM activity messages can be monitored.

The CHECK mode 234 actually serves two functions: system check prior to using the PM and calibration check. Both functions operate together and appear the same to the ordinary user. The first action 240 is to test the battery voltage. If the voltage is too low, an alarm is given and all LEDs are turned off at 244. If the voltage is good enough for 4 hours of operation, the program will test the diaphragm sound transducer 186 and the LEDs 164, 208, 210, 212 at 242 by sounding an alarm and turning on each LED on in sequence at one second intervals. This allows for a visual check of the LEDs and an aural check of the alarm.

The LEDs 164 and 208-212 will flash with each heart beat if the sensor module 10 is connected and a good EKG signal is being obtained. If the sensor module 10 is disconnected, the lights will remain steady. If the EKG signal is poor, either the LEDs will not flash or the flashing will be very irregular (not a rhythmic beat like the heart). While in CHECK, the LEDs will continue to light in sequence and the battery will be checked every 15 seconds until the command buttons 204 and 206 are actuated at 246, at which time the program returns to the IDLE mode 232. It then waits to read the function switch 118 at 248 with the next activation of Command. If the battery voltage drops below the starting level criterion, the alarm sounds and all LEDs go off.

While in the CHECK mode 234, a calibration check can be made. This would be a bench test during which a printer or computer terminal is connected to the PM through the RS232 port 108. The temperature sensor 14 is placed in a stirred water bath that is between 35 and 40 ° C (95 and 104 ° F) and measured with a thermometer with an accuracy of ±0.05 ° C(0.1 ° F). A pulse generator is attached through a voltage divider to the electrode clips 12 so that a 2 millivolt pulse (positive to the left electrode) is delivered. Every 15 seconds, pulse rate in pulses per minute, disk temperature in degrees-C times 10 (e.g., 375 means 37.5 ° C) and battery voltages times 10 (e.g., 77 means 7.7 volts) are sent out on the RS232 port 108. If the temperature readings are within 0.05 ° C and the pulse rate within 2 pulses per minute, the sensor module 10 and monitor module 100 are working correctly. The calibration check can proceed if the low battery signal is given, but erroneous temperature readings may occur if battery voltage is less than 7.1 volts. The calibration check is ended by pushing the command buttons 204 and 206, which returns the program to the IDLE mode 232.

When the function switch 118 reads RUN at the Command, the PM enters the operations mode. At this point, only the Green LEDs 212 are on, and Yellow and Red are deactivated. In this mode, the PM monitors physiological state. There are four main activities in the RUN mode: initialization 248, after first 30 seconds of each minute 250, after second 30 seconds of each minute 252 and recovery heart rate routine 254. Initialization 248 is the first activity, and is performed only when entering the RUN mode 236 from the IDLE mode 232. The Age and Clothing switches are read. Depending on switch positions, the relevant thresholds for Green-to-Yellow (Warning), Yellow-to Green (Reset), and Yellow-to-Red (Action) for disk temperature and moving-time-average heart rates (MTAs) are loaded into the data array. All heart rate values in a delay stack and the MTAs are set to 75, the assumed value for resting HR. The timer and heart beat counter are set to zero.

At the end of the first half of every minute following the start in RUN mode, the battery voltage and heart rate counter are read at 256. The counter is immediately reset to zero, and the heart beats are doubled to give the rate in beats per minute (bpm). The next step is to perform a quality check on the heart rate. If the value is between 40 and 220 bpm and the increase from the previous reading is less than 30%, the heart rate is recorded as read. If HR<40 or HR>220, then the value is out of acceptable bounds. This means the rate is physiologically too high or too low and is due to a poor EKG signal. In either case, HR is taken as the preceding recorded value (the one taken from 60-30 seconds preceding the current time). An increase greater than 30% is not likely and may be due to some spurious pulses from the EKG electrodes. In this case, the increase is limited to 30%. This will not have any real effect on the MTA's if it is a true increase because HR will be quick to reach the new value. If the increase is artificial, HR returns to the correct value and the effect of the increased value is contained, for a small effect on the MTAs.

A variable called the net number variable (HR error index) is increased by one for each questionable reading and reduced by one for each good reading, but is not allowed to go less than zero or more than 7. If there are a net number of 7 questionable readings, where the 30 second HR values were adjusted, an alarm is given. In the worst case, this means there are 3 complete minutes of questionable data for HR. The alert for inaccurate HR data is the aural alarm and all LEDs turning on.

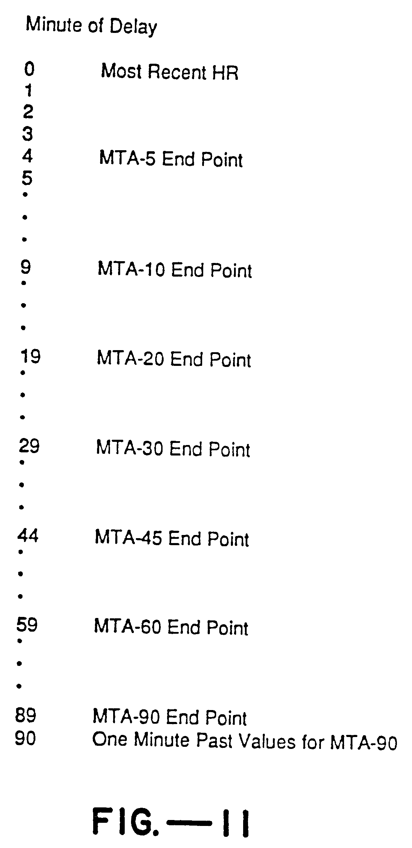

The next step is to shift the delay stack by one minute at 258. The delay stack is illustrated in Figure 11. The first value is for the current minute, the second for the preceding minute, third for two minutes preceding, and so on until the last value represents the 90th preceding the current time. Remember that a resting value of 75 is assumed at the start, and therefore the stack is prefilled with 75s. The stack is shifted so that, first the value in 89 replaces 90, then 88 replaces 89, and so on until the last current value at 0 is moved to 1. The stack represents the time course of HR for the past 90 minutes, with the 91st minute thrown away. At this point, the rate for the first half minute is placed at 0. The program now checks to see if a recovery is in progress. This is discussed below.

The battery voltage is checked. If it is less than or equal to 7.0 volts for three consecutive readings, an alarm is sounded and all LEDs are cleared (turned off). The poor HR alert overrides. The device continues to operate so that data is collected, but there will not be a continuous display of alert status. At this point, the temperature readings may not be accurate, but the failure mode is toward increasing values so it is protective.

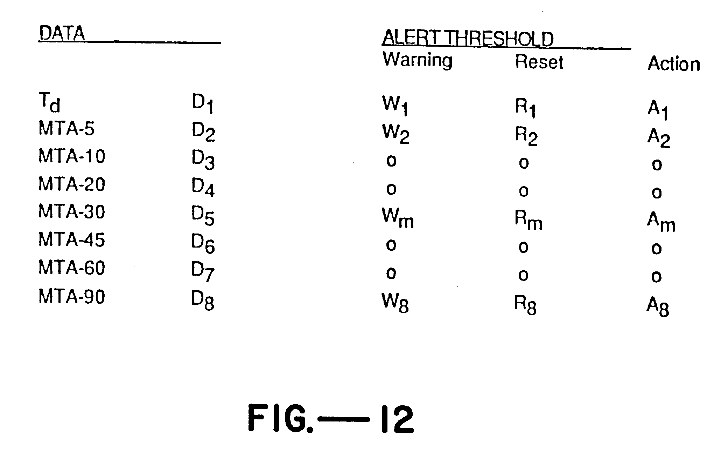

In the same manner as described above, the heart rate counter is read and reset at 260, and a quality check on HR is made. The HR value for the second 30 seconds is averaged with the value for the first 30 seconds, which gives the total count for the past minute. This value is placed in location 0 of Figure 11. The next step is to calculate the MTAs at 262. MTA-5 is the moving-time-average for 5 minutes, and would be the sum of values from 0 to 4 in Figure 11 divided by 5. MTA-10 would be the sum of values 0 to 9 divided by 10. As a computational shortcut, the sums are maintained in separate variables, the newest value (always location 0 is added to the total) and the oldest value is subtracted. For MTA-5, the oldest value would be at location 5; for MTA-10 at 10, and so on. Once the MTAs have been updated, the alert status can be determined at 264. The physiological states, i.e. skin temperature Ta, and moving time average heart rates MTA-5 to MTA-90, are represented by the data values D1 through D8 in Figure 12. The respective threshold values are represented in similar fashion with W being the Warning (Yellow) level, R being the Reset (return to Green) level and A being the Action (Red) level.

The following decision process is used in the program:

```
1. If YELLOW iş current alert state, should PM
   be set to GREEN?
     *for m= 1 to 8: if Dm <Rm then WFm = 0
                     (WFm is a flag to indicate a change to
                     GREEN)
     *WFT = ΣWFm:  if WFT =0 then set GREEN
2. If YELLOW is current alert state, should PM
   be set to RED?
     * for m=1 to 8: if Dm>Am then set RED
3. If GREEN is current alert state, should PM be
set to YELLOW?
     *for m = 1 to 8: if Dm>Wm, then WFm = 1
     (WFm is a flag to indicate a change to
     YELLOW)
     *WFT=ΣWFm: if WFT>0 then set alert state to
     YELLOW
4. Wait to next minute:  collect new data and go
   to 1
```

If there was a change in alert status due to YELLOW to GREEN, YELLOW to RED, GREEN to YELLOW, an alarm sound is given (steady tone for 3 seconds) and the appropriate LED is selected. This will occur even if the battery is too low for continuous operation. In this case, the LED will stay on for 30 seconds. The physiological data and PM status for the preceding minute are stored in the memory. These data can be retrieved in the DUMP mode 238 (see below). If the PM device is attached to a printer or terminal (unlikely for field use), a printout is provided for every minute of the time, current HR, disk temperature (x10), alert status and LED status (0-all LEDs off, 1-GREEN, 2-YELLOW, 3-RED, 4-all LEDs on), heart rate quality status and battery voltage (x10) at 266. This feature is provided for laboratory tests and demonstrations. The program then returns to wait until the first 30 seconds of the next minute are complete.

The recovery routine 254 can be invoked at any time during the RUN mode by activating the command buttons 204 and 206. When the Command is requested, the function switch 118 is checked. If the selected function is RUN, then recovery routine 254 is requested. If a second request is made before the first is serviced, then the first one is ignored and recovery is reinitialized. The first step is to synchronize the request to the sampling period by moving it to the nearest one-half minute on the PM clock. By doing this, the calculation of $P_1$, $P_2$, and $P_3$ (see Section 2) is off by ±15 seconds in the worst case.

The recovery routine follows these steps:
1. Record heart beats for the following intervals:
     *30 to 60 sec. after request ($P_1$ = total beats x 2)
     *90 to 120 sec. after request ($P_2$ = total beats x 2)
     *150 to 180 sec. after request ($P_3$ = total beats x 2)
2. If $P_3 < 90$ then
     *sound alarm
     *flash GREEN
     *go to 5
3. If $P_3 < 120$ and ($P_1$-$P_3$ >10) then
     *sound alarm

*flash YELLOW for 15 seconds

*go to 5

4. Sound alarm and flash RED for 15 seconds

5. Return LEDs to previous status and leave recovery routine

The values for $P_1$, $P_2$, and $P_3$ are taken at one minute intervals and represent 30 second counts for the second half of the three recovery minutes, as called for by the procedure. When all three values have been obtained, the recovery status is presented to the user. First, the aural alert is sounded, then the appropriate LED is flashed on for 1 second and off for 1 second for 15 seconds. The LEDs are then returned to their previous state. The result of the recovery routine is sent out on the RS232 port 108 and stored in the DUMP memory.

The RUN mode can be terminated by changing the function switch from RUN to either CHECK or DUMP and pushing the command buttons 204 and 206. Program control will always go to the CHECK mode. If the RUN mode is entered with the Demo/Dump Format switch 120 in the CW position, a demonstration of the PM alerts is given. In 5-second intervals the PM will go through the following alert sequence: Green-Yellow-Green-Yellow-Red. At the Red LED, the program waits ten seconds for the command buttons 204 and 206 to demonstrate a Yellow recovery alert. The PM will return to the IDLE mode if the command buttons 204 and 206 are activated with a Green or Yellow LED on or 10 seconds after the Red recovery alert.

The DUMP mode 238 permits the extraction of information on both physiological and PM performance at the end of a PM session. To preserve the stored data, the user must

1. Select CHECK or DUMP on the function switch 118 to exit RUN

2. Activate the command buttons 204 and 206

3. Attach auxiliary battery or do not disconnect Alert module

4. Remove Monitor module from person

5. Connect a printer or computer terminal to the RS232 port 108

6. Select DUMP on the function switch 118

7. Activate the command buttons 204 and 206

The aural alarm is given and the LEDs go to the Yellow and Red on (Green off). The data will be sent at 270 in the selected format, at the selected baud rate, 8 bits/word, 1 stop bit, and no parity. If the receiving unit does not support the XON/XOFF protocol, the results may become garbled due to buffer overflow.

The baud rate on the communications port can be changed using the Baud Rate switch 122. The change becomes effective immediately upon entering the DUMP mode. The Demo/Dump Format switch is also read and acted on when the DUMP mode is entered.

There are basically two data types. One is the principal data that are stored at one minute intervals. These include the seven MTAs, so that the calculated averages (values used to determine alert status) are available. (The MTA output can be omitted as an option selected with Demo/Dump Format switch 120.) Flag Status is an eight digit number where each digit represents a working variable (disk temperature and MTAs) that indicates individual alert levels. HR error index is the net number of questionable 30 second heart rate values (labelled QC for quality check). The last entry is battery voltage times 10. The second type of output is recovery data, which gives the report time, recovery status, and the three recovery heart rates.

The program will automatically return to the IDLE mode 232 at the end of the download. The download can also be stopped by activating the command buttons 204 and 206, which returns the program to the IDLE mode.

Four special purpose routines were written into the PM program to aid development. They are stored at the end. To use these routines, the program must be interrupted by sending (entering or typing) from a terminal a control-A(__A) in the IDLE mode. At this point control passes to TTBASIC monitor.

Disk temperature calibration factors, starting battery voltage criterion and serial number can be changed by executing "GO TO 21000." The routine will ask for the intercept ($°C$) and then the slope ($°C$/unit), each times 1000. It then asks for the battery voltage times 10 that is the minimum required for PM operations. These values are then stored in nonvolatile memory and printed out for verification.

Run counter and serial number can be reset to a new number by executing a "GOTO 21100." A deep sleep routine at line 30000 was used to conserve battery life without removing power to the PM. It is useful for the long term storage of PM data. A/D converter readings for all 8 channels and battery voltage are provided by starting execution at line 31000. It updates at 3 second intervals. Control can be returned to the PM by either removing power and restarting, or by executing a "RUN."

It should now be readily apparent to those skilled in the art that a novel personal monitor and process for heat and work stress has been provided. There are three novel features: First, a novel temperature sensor especially adapted for use in the monitor and process capable of achieving the stated objects of the invention has been provided. Second, the process uses multiple moving averages in time to examine heart rate patterns. Thresholds for each of the multiple moving time averages are used to mark the point of excessive physiological demand. Third, a commonly accepted heart rate recovery routine has been automated within the PM. The personal monitor and process measures physiological responses to work and heat stress and provides recommendations to the user in an industrial environment which will allow the user to limit his or her exposure to the physiological stress in an informed manner. The monitor and process neither provides the user with a multitude of inappropriate warnings nor allows the user to exceed safe limits without providing appropriate warnings. The novel temperature sensor monitors of core body temperature in a consistent and reliable manner.

It should further be apparent to those skilled in the art that various modifications in form and details of the invention as shown and described may be made. For example, while the hardware has been implemented with commercially available standard integrated circuits in three modules, it could be made substantially smaller through the use of custom integrated circuits, resulting in one or two smaller modules. An infrared or radio transmission link could be used between the modules and/or sensors. With such links, the system could also telemeter out to a supervisory unit. The switches could be replaced with a different user interface. The sensor electrodes could be implemented as a two electrode system, rather than a three electrode system by providing a single EKG active electrode and using the temperature sensor electrode as the EKG passive electrode as well. The system can be used for other applications, such as for sports and/or rehabilitation training, and as a physiological data logger. For the sports and rehabilitation application, the system could be modified to provide a programmed training regimen that varies in time and provides both a minimum and maximum alert, so that the user obtains a desired level and pacing of exercise, without exceeding safe limits. While the temperature sensor is shown as particularly configured for monitoring core body temperature, it could be adapted for monitoring the temperature of a wide variety of other objects as well.

## Claims

1. A personal heart rate monitor comprising a heart beat sensor (12, 62) producing output electrical signals indicating a user's heart beats, means (124) for storing heart beat information corresponding to the heart beat signals produced, information processing means (126) connected to receive the heart beat information from said storing means (124), said information processing means (126) being configured to analyse the heart beat information to obtain a physiological demand and to compare the obtained physiological demand against a stored physiological demand limit, and means (200) for providing an indication to the user when the stored physiological demand limit has been exceeded, said information processing means (126) being configured and connected to control operation of said indication providing means (200), characterised in that the information processing means (126) under software control analyses the heart beat information incrementally over predetermined time intervals through moving time averages with a plurality of different time bases by incrementing the time base of the moving time averages.

2. A personal heart rate monitor according to claim 1, characterised in that an insulated skin temperature sensor (14) is provided to produce output electrical signals at least approximately indicating a user's body core temperature, said information processing means (126) being connected to receive body core temperature information corresponding to the body core temperature output electrical signals, said information processing means (126) further being configured to compare the body core temperature information against a stored body core temperature limit, and said information processing means (126) further being configured to cause said means (200) for providing the indication to the user to provide the indication when the user's body core temperature exceeds the stored body core temperature limit.

3. A personal monitor according to claim 2, characterised in that said insulated skin temperature sensor (14) comprises a thermally and electrically conducting member (26) having a surface configured to make direct contact with the user's skin, a device (38) thermally connected to said thermally and electrically conducting member (26) for producing an output electrical signal which is a function of temperature and a sufficient body (22) of thermally insulating material surrounding a remainder of said thermally and electrically conducting member (26) to provide the output electrical signal indicating skin temperature as an approximation of body core temperature.

EP 0 319 551 B1

**4.** A personal monitor according to claim 3, characterised in that said thermally and electrically conducting member (26) is connected as a ground electrode for said heart beat sensor.

**5.** A personal monitor according to any one of claims 2, 3, and 4, characterised by an input means (114) for providing a user clothing type selection to said information processing means (126) and in that said information processing means is further configured to select the body temperature limit based on the user clothing type selection for comparison with the body temperature information.

**6.** A personal monitor according to any preceding claim, characterised by an input means (116) for providing a user age selection to said information processing means (126) and in that said information processing means (126) is further configured to select the stored physiological demand limit for the comparison based on the user age selection.

**7.** A personal monitor according to any preceding claim, characterised by an input means (118) for user selection of an operation mode, and in that said information processing means (126) is further configured to record user heart beat rates for a plurality of intervals and to compare the user hear beat rate for at least one of the intervals in response to user selection of an operation mode and to provide an indication of the comparison to the user.

**8.** A personal monitor according to claim 7, characterised in that said information processing means (126) is further configured to compare the user heart beat rate for a last of the intervals against a predetermined limit and to provide an indication of the comparison to the user.

**9.** A personal monitor according to claim 3 or 4, characterised in that said thermally and electrically conducting member (26) is a copper member.

**10.** A personal monitor according to claim 2, characterised in that the temperature sensor (14) comprises a thermally and electrically conducting member (26) having a bare, outwardly facing, unbroken surface configured and positioned to make direct contact with the skin of a person whose temperature is to be measured, a temperature sensitive solid state device (38) thermally and electrically directly connected to an opposite surface of said thermally and electrically conducting member (26) for producing an output electrical signal which is a function of temperature and a sufficient body (22) of thermally insulating material surrounding a remainder of said thermally and electrically conducting member to provide the output electrical signal indicating insulated skin temperature as an approximation of the person's body core temperature, said thermally and electrically conducting member (26) comprising both a thermal contact for the person's skin and a reference electrode of said temperature sensor.

**11.** A personal monitor according to claim 10, characterised in that said body (22) of thermally insulating material comprises a foamed plastic.

**12.** A personal monitor according to claim 10 or 11, characterised in that said thermally and electrically conducting member (26) is a copper member.

**13.** A process for monitoring the heart rate of an individual which comprises measuring the individual's heart beats, analyzing the heart beat information to obtain a physiological demand, comparing the obtained physiological demand against a predetermined physiological demand limit, and providing an indication to the individual when the predetermined physiological demand limit has been exceeded, characterised by analyzing the heart beat information incrementally over predetermined time intervals through moving time averages with a plurality of different time bases by incrementing the time base of the moving time averages.

**14.** A process according to claim 13 characterised by additionally, for the purpose of monitoring heat and work stress, measuring the individual's insulated skin temperature as an approximation of body core temperature during the predetermined time intervals, comparing the measured approximation of body core temperature against a predetermined temperature limit and providing an indication to the individual when the measured approximation of body core temperature exceeds the predetermined temperature limit.

18

EP 0 319 551 B1

**15.** A process according to claim 14, characterised in that the approximation of body core temperature is measured by contacting the individual's skin with a surface configured to make direct contact with the user's skin of a thermally and electrically conducting member (26), insulating a remainder of the thermally and electrically conducting member (26), and producing an output electrical signal which is a function of temperature of the thermally and electrically conducting member (26).

**16.** A process according to claim 14 or 15, characterised by the step of selecting the predetermined temperature limit based on a clothing type for the individual.

**17.** A process according to claim 15, characterised in that the thermally and electrically conducting member (26) is a copper member.

**18.** A process according to any one of claims 13 to 17, characterised by the step of selecting the physiological demand limit based on the individual's age.

**Patentansprüche**

**1.** Personen-Herzschlag-Überwachungsgerät mit einem Herzschlagsensor (12, 62), der die Herzschläge eines Benutzers anzeigende elektrische Ausgangssignale erzeugt, einer Einrichtung (124) zum Speichern den erzeugten Herzschlagsignalen entsprechender Herzschlaginformation, einer die Herzschlaginformation von der Speichereinrichtung (124) erhaltenden Informationsverarbeitungseinrichtung (126), die so aufgebaut ist, daß sie die Herzschlaginformation analysiert, so daß eine physiologische Anforderung erhalten wird, und die erhaltene physiologische Anforderung mit einer gespeicherten physiologischen Anforderungsgrenze vergleicht, und einer Einrichtung (200) zur Bereitstellung einer Anzeige für den Benutzer, wenn die gespeicherte physiologische Anforderungsgrenze überschritten wurde, wobei die Informationsverarbeitungseinrichtung (126) so aufgebaut und angeschlossen ist, daß sie den Betrieb der Anzeige-Bereitstellungseinrichtung (200) steuert, dadurch gekennzeichnet, daß die Informationsverarbeitungseinrichtung (126) softwaregesteuert die Herzschlaginformation inkrementell über vorbestimmte Zeitintervalle durch bewegliche Zeitmittel mit mehreren unterschiedlichen Zeitbasen durch Inkrementieren der Zeitbasis der bewegenden Zeitmittel analysiert.

**2.** Personen-Herzschlag-Überwachungsgerät nach Anspruch 1, gekennzeichnet durch einen isolierten Hauttemperaturfühler (14), dessen elektrische Ausgangssignale wenigstens ungefähr die Körper-Innentemperatur eines Benutzers anzeigen, wobei die Informationsverarbeitungseinrichtung (126) so angeschlossen ist, daß sie den elektrischen Körper-Innentemperatur-Ausgangssignalen entsprechende Körpertemperaturinformation erhält, wobei die Informationsverarbeitungseinrichtung (126) ferner so aufgebaut ist, daß die Körper-Innentemperaturinformation mit einer gespeicherten Körper-Innentemperaturgrenze verglichen wird, und wobei die Informationsverarbeitungseinrichtung (126) ferner so aufgebaut ist, daß die Einrichtung (200) zur Bereitstellung der Anzeige für den Benutzer dem Benutzer eine Anzeige bereitstellt, wenn seine Körper-Innentemperatur die gespeicherte Körper-Innentemperaturgrenze übersteigt.

**3.** Personen-Überwachungsgerät nach Anspruch 2, dadurch gekennzeichnet, daß der isolierte Hauttemperaturfühler (14) einen thermisch und elektrisch leitfähigen Teil (26) enthält, dessen Oberfläche so ausgebildet ist, daß er die Haut des Benutzers direkt berührt, sowie ferner eine Einrichtung (38), die thermisch mit dem thermisch und elektrisch leitfähigen Teil (26) verbunden ist und ein elektrisches Ausgangssignal erzeugt, das eine Funktion der Temperatur ist, und einen ausreichenden Körper (22) aus thermisch isolierendem Material, der einen Rest des thermisch und elektrisch leitfähigen Teils (26) umgibt, so daß elektrische Ausgangssignale bereitgestellt werden, die die Hauttemperatur als Annäherung an die Körper-Innentemperatur anzeigen.

**4.** Personen-Überwachungsgerät nach Anspruch 3, dadurch gekennzeichnet, daß der thermisch und elektrisch leitfähige Teil (26) als Masseelektrode für den Herzschlagsensor angeschlossen ist.

**5.** Personen-Überwachungsgerät nach einem der Ansprüche 2, 3 und 4, gekennzeichnet durch eine Eingabeeinrichtung (114) zur Bereitstellung einer Kleidungsart-Auswahl des Benutzers für die Informationsverarbeitungseinrichtung (126), sowie ferner dadurch, daß die Informationsverarbeitungseinrichtung weiter so aufgebaut ist, daß die Körpertemperaturgrenze auf der Grundlage der Kleidungsart-Auswahl

19

des Benutzers zum Vergleich mit der Körpertemperaturinformation gewählt wird.

6. Personen-Überwachungsgerät nach einem der vorstehenden Ansprüche, gekennzeichnet durch eine Eingabeeinrichtung (116) zur Bereitstellung einer Alterswahl des Benutzers für die Informationsverarbeitungseinrichtung (126), und ferner dadurch, daß die Informationsverarbeitungseinrichtung (126) so aufgebaut ist, daß die gespeicherte physiologische Anforderungsgrenze zum Vergleich auf der Basis der Alterswahl des Benutzers gewählt wird.

7. Personen-Überwachungsgerät nach einem der vorstehenden Ansprüche, gekennzeichnet durch eine Eingabeeinrichtung (118) zur Wahl eines Betriebsmodus durch den Benutzer und ferner dadurch, daß die Informationsverarbeitungseinrichtung (126) so ausgebildet ist, daß für mehrere Intervalle die Herzschlagfrequenz des Benutzers aufgezeichnet und die Herzschlagfrequenz des Benutzers entsprechend der Wahl des Betriebsmodus durch den Benutzer für wenigstens einen der Intervalle verglichen und eine Anzeige des Vergleichs für den Benutzer bereitgestellt wird.

8. Personen-Überwachungsgerät nach Anspruch 7, dadurch gekennzeichnet, daß die Informationsverarbeitungseinrichtung (126) so ausgebildet ist, daß die Herzschlagfrequenz des Benutzers für einen letzten der Intervalle mit einer vorbestimmten Grenze verglichen und eine Anzeige des Vergleichs für den Benutzer bereitgestellt wird.

9. Personen-Überwachungsgerät nach Anspruch 3 oder 4, dadurch gekennzeichnet, daß der thermisch und elektrisch leitfähige Teil (26) ein Kupferteil ist.

10. Personen-Überwachungsgerät nach Anspruch 2, dadurch gekennzeichnet, daß der Temperaturfühler (14) einen thermisch und elektrisch leitfähigen Teil (26) mit einer freiliegenden, nach außen weisenden, ungebrochenen Oberfläche aufweist, die so ausgebildet und angeordnet ist, daß sie die Haut einer Person, deren Temperatur gemessen werden soll, direkt berührt, sowie ferner eine temperaturempfindliche Festkörpereinrichtung (38), die thermisch und elektrisch direkt mit einer gegenüberliegenden Fläche des thermisch und elektrisch leitfähigen Teils (26) verbunden ist, zum Erzeugen eines elektrischen Ausgangssignals, das eine Funktion der Temperatur ist, und einen ausreichenden Körper (22) aus thermisch isolierendem Material, der einen Rest des thermisch und elektrisch leitfähigen Teils umgibt, so daß ein elektrisches Ausgangssignal bereitgestellt wird, das die isolierte Hauttemperatur als Annäherung an die Körper-Innentemperatur der Person anzeigt, wobei der thermisch und elektrisch leitfähige Teil (26) einen thermischen Kontakt für die Haut der Person und eine Bezugselektrode des Temperaturfühlers aufweist.

11. Personen-Überwachungsgerät nach Anspruch 10, dadurch gekennzeichnet, daß der Körper (22) aus thermisch isolierendem Material geschäumten Kunststoff enthält.

12. Personen-Überwachungsgerät nach Anspruch 10 oder 11, dadurch gekennzeichnet, daß der thermisch und elektrisch leitfähige Teil (26) ein Kupferteil ist.

13. Verfahren zur Überwachung des Herzschlags einer Person, das umfaßt: Messen der Herzschläge der Person, Analysieren der Herzschlaginformation, um eine physiologische Anforderung zu erhalten, Vergleichen der erhaltenen physiologischen Anforderung mit einer vorbestimmten physiologischen Anforderungsgrenze und Bereitstellen einer Anzeige für die Person, wenn die vorbestimmte physiologische Anforderungsgrenze überschritten wurde, dadurch gekennzeichnet, daß die Herzschlaginformation inkrementell über vorbestimmte Zeitintervalle durch bewegende Zeitmittel mit mehreren unterschiedlichen Zeitbasen durch Inkrementieren der Zeitbasis der bewegenden Zeitmittel analysiert wird.

14. Verfahren nach Anspruch 13, dadurch gekennzeichnet, daß ferner zur Überwachung von Wärme und Arbeitsbeanspruchung die isolierte Hauttemperatur der Person als Annäherung an die Körper-Innentemperatur während der vorbestimmten Zeitintervalle gemessen wird, wobei die gemessene Annäherung an die Körper-Innentemperatur mit einer vorbestimmten Temperaturgrenze verglichen und eine Anzeige für die Person bereitgestellt wird, wenn die gemessene Annäherung an die Körper-Innentemperatur die vorbestimmte Temperaturgrenze überschreitet.

20

**15.** Verfahren nach Anspruch 14, dadurch gekennzeichnet, daß die Annäherung an die Körper-Innentemperatur durch die Berührung der Haut der Person mit einer Oberfläche gemessen wird, die so ausgebildet ist, daß ein thermisch und elektrisch leitfähiger Teil (26) die Haut des Benutzers direkt berührt, wobei ein Rest des thermisch und elektrisch leitfähigen Teils (26) isoliert und ein elektrisches Ausgangssignal erzeugt wird, das eine Funktion der Temperatur des thermisch und elektrisch leitfähigen Teils (26) ist.

**16.** Verfahren nach Anspruch 14 oder 15, gekennzeichnet durch den Schritt der Wahl einer vorbestimmten Temperaturgrenze auf der Grundlage einer Kleidungsart des Benutzers.

**17.** Verfahren nach Anspruch 15, dadurch gekennzeichnet, daß der thermisch und elektrisch leitfähige Teil (26) ein Kupferteil ist.

**18.** Verfahren nach einem der Ansprüche 13 bis 17, gekennzeichnet durch den Schritt der Wahl der physiologischen Anforderungsgrenze auf der Grundlage des Alters des Benutzers.

**Revendications**

**1.** Moniteur personnel de rythme cardiaque comprenant un détecteur (12, 62) des battements cardiaques produisant des signaux électriques de sortie indiquant les battements cardiaques de l'utilisateur, des moyens (124) pour stocker l'information relative aux battements cardiaques correspondant aux signaux de battements cardiaques produits, des moyens (126) de traitement de l'information connectés pour recevoir l'information relative aux battements cardiaques en provenance desdits moyens (124) de stockage, lesdits moyens (126) de traitement de l'information étant configurés pour analyser l'information relative aux battements cardiaques afin d'obtenir une demande physiologique et pour comparer la demande physiologique obtenue et une limite de demande physiologique stockée, et des moyens (200) pour fournir une indication à l'utilisateur lorsque la limite de demande physiologique stockée a été dépassée, lesdits moyens (126) de traitement de l'information étant configurés et connectés pour commander le fonctionnement desdits moyens (200) de fourniture d'indication, caractérisé en ce que les moyens (126) de traitement de l'information sous commande de logiciel analysent l'information relative aux battements cardiaques de manière incrémentielle pendant des intervalles de temps prédéterminés par le déplacement de moyennes de temps avec une pluralité de bases de temps différentes en incrémentant la base de temps des moyennes de temps qui se déplacent.

**2.** Moniteur personnel de rythme cardiaque selon la revendication 1, caractérisé en ce qu'un détecteur (14) de la température de la peau isolée est prévu pour produire des signaux électriques de sortie indiquant au moins approximativement la température centrale du corps d'un utilisateur, lesdits moyens (126) de traitement de l'information étant connectés pour recevoir l'information relative à la température centrale du corps correspondant aux signaux électriques de sortie de température centrale du corps, lesdits moyens (126) de traitement de l'information étant en outre configurés pour comparer l'information relative à la température centrale du corps avec une limite de température centrale du corps stockée, et lesdits moyens (126) de traitement de l'information étant en outre configurés pour conduire lesdits moyens (200) à fournir l'indication à l'utilisateur afin d'indiquer lorsque la température centrale du corps de l'utilisateur excède la limite de température centrale du corps stockée.

**3.** Moniteur personnel selon la revendication 2, caractérisé en ce que ledit détecteur (14) de température de la peau isolée comporte un élément (26) conducteur électriquement et thermiquement ayant une surface configurée pour être en contact direct avec la peau de l'utilisateur, un dispositif (38) connecté thermiquement audit élément (26) conducteur thermiquement et électriquement pour produire un signal électrique de sortie qui est une fonction de la température et un corps suffisant (22) en matériau isolant thermiquement entourant le reste dudit élément (26) conducteur thermiquement et électriquement pour délivrer le signal électrique de sortie indiquant la température de la peau comme une approximation de la température centrale du corps.

**4.** Moniteur personnel selon la revendication 3, caractérisé en ce que ledit élément (26) conducteur thermiquement et électriquement est connecté comme une électrode à la masse pour ledit détecteur de battements cardiaques.

**5.** Moniteur personnel selon l'une quelconque des revendications 2, 3 et 4, caractérisé par un moyen d'entrée (114) pour fournir une sélection du type vêtement de l'utilisateur auxdits moyens (126) de traitement de l'information et en ce que lesdits moyens de traitement de l'information sont en outre configurés pour sélectionner la limite de température du corps en fonction de la sélection du type de vêtement de l'utilisateur pour la comparaison avec l'information de la température du corps.

**6.** Moniteur personnel selon l'une quelconque des revendications précédentes, caractérisé par des moyens d'entrée (116) pour fournir une sélection de l'âge de l'utilisateur auxdits moyens (126) de traitement de l'information, et en ce que lesdits moyens (126) de traitement de l'information sont en outre configurés pour sélectionner la limite de demande physiologique stockée pour la comparaison en fonction de la sélection de l'âge de l'utilisateur.

**7.** Moniteur personnel selon l'une quelconque des revendications précédentes, caractérisé par des moyens d'entrée (118) pour la sélection par l'utilisateur d'un mode de fonctionnement, et en ce que lesdits moyens (126) de traitement de l'information sont en outre configurés pour enregistrer les rythmes des battements cardiaques pendant une pluralité d'intervalles et pour comparer le rythme des battements cardiaques pendant au moins l'un des intervalles en réponse à la sélection par l'utilisateur d'un mode de fonctionnement et pour fournir une indication de la comparaison à l'utilisateur.

**8.** Moniteur personnel selon la revendication 7, caractérisé en ce que lesdits moyens (126) de traitement de l'information sont en outre configurés pour comparer le rythme des battements cardiaques de l'utilisateur pour un dernier des intervalles avec une limite prédéterminée et pour fournir une indication de la comparaison à l'utilisateur.

**9.** Moniteur personnel selon la revendication 3 ou 4, caractérisé en ce que ledit élément (26) conducteur thermiquement et électriquement est un élément en cuivre.

**10.** Moniteur personnel selon la revendication 2, caractérisé en ce que le détecteur de température (14) comporte un élément (26) conducteur thermiquement et électriquement ayant une suface nue, orientée vers l'extérieur, non brisée, configurée et mise en contact direct avec la peau d'une personne dont la température est à mesurer, un dispositif (38) semiconducteur sensible à la température connecté thermiquement et électriquement de façon directe à une surface opposée dudit élément (26) conducteur thermiquement et électriquement pour produire un signal électrique de sortie qui est une fonction de la température et un corps suffisant (22) en un matériau isolant thermiquement entourant le reste dudit élément conducteur thermiquement et électriquement pour fournir le signal électrique de sortie indiquant la température de la peau isolée comme une approximation de la température centrale du corps d'une personne, ledit élément (26) conducteur thermiquement et électriquement comprenant à la fois un contact thermique pour la peau de la personne et une électrode de référence dudit détecteur de température.

**11.** Moniteur personnel selon la revendication 10, caractérisé en ce que ledit corps (22) en matériau isolant thermiquement comporte une mousse de matière plastique.

**12.** Moniteur personnel selon la revendication 10 ou 11, caractérisé en ce que ledit élément (26) conducteur thermiquement et électriquement est un élément en cuivre.

**13.** Procédé de contrôle du rythme cardiaque d'un individu qui consiste à mesurer les battements cardiaques de l'individu, à analyser l'information relative aux battements cardiaques pour obtenir une demande physiologique, à comparer la demande physiologique obtenue avec une limite de demande physiologique prédéterminée, et à fournir une indication à l'individu lorsque la limite de demande physiologique prédéterminée a été dépassée, caractérisé par l'analyse de l'information relative aux battements cardiaques de manière incrémentielle pendant des intervalles de temps prédéterminés en déplaçant des moyennes de temps avec une pluralité de bases de temps différentes en incrémentant la base de temps des moyennes de temps qui se déplacent.

**14.** Procédé selon la revendication 13, caractérisé en outre, dans le but de contrôler la fatigue due au travail et à la chaleur, par la mesure de la température de la peau isolée de l'individu comme une approximation de la température centrale du corps au cours des intervalles de temps prédéterminés, la comparaison de l'approximation mesurée de la température centrale du corps avec une limite de température prédéterminée et la fourniture d'une indication à l'individu lorsque l'approximation mesurée de la température centrale du corps excède la limite de température prédéterminée.

15. Procédé selon la revendication 14, caractérisé en ce que l'approximation de la température centrale du corps est mesurée en mettant la peau de l'individu en contact avec une surface configurée pour être en contact direct avec la peau de l'utilisateur d'un élément (26) conducteur thermiquement et électriquement, en isolant le reste de l'élément (26) conducteur thermiquement et électriquement, et en produisant un signal électrique de sortie qui est une fonction de la température de l'élément (26) conducteur thermiquement et électriquement.

16. Procédé selon la revendication 14 ou 15, caractérisé par l'étape de sélection de la limite de température prédéterminée basée sur un type de vêtement pour l'individu.

17. Procédé selon la revendication 15, caractérisé en ce que l'élément (26) conducteur thermiquement et électriquement est un élément en cuivre.

18. Procédé selon l'une quelconque des revendications 13 à 17, caractérisé par l'étape de sélection de la limite de demande physiologique basée sur l'âge de l'individu.

FIG.—1

FIG.—2

FIG.-3

EP 0 319 551 B1

FIG.—4

FIG.-5

FIG.—6

EP 0 319 551 B1

FIG.—7

EP 0 319 551 B1

FIG.-8

FIG.-9

FIG.—10

Minute of Delay

| | |
|---|---|
| 0 | Most Recent HR |
| 1 | |
| 2 | |
| 3 | |
| 4 | MTA-5 End Point |
| 5 | |
| • | |
| • | |
| • | |
| 9 | MTA-10 End Point |
| • | |
| • | |
| • | |
| 19 | MTA-20 End Point |
| • | |
| • | |
| • | |
| 29 | MTA-30 End Point |
| • | |
| • | |
| • | |
| 44 | MTA-45 End Point |
| • | |
| • | |
| • | |
| 59 | MTA-60 End Point |
| • | |
| • | |
| • | |
| 89 | MTA-90 End Point |
| 90 | One Minute Past Values for MTA-90 |

# FIG. — 11

| DATA | | ALERT THRESHOLD | | |
| --- | --- | --- | --- | --- |
| | | Warning | Reset | Action |
| $T_d$ | $D_1$ | $W_1$ | $R_1$ | $A_1$ |
| MTA-5 | $D_2$ | $W_2$ | $R_2$ | $A_2$ |
| MTA-10 | $D_3$ | o | o | o |
| MTA-20 | $D_4$ | o | o | o |
| MTA-30 | $D_5$ | $W_m$ | $R_m$ | $A_m$ |
| MTA-45 | $D_6$ | o | o | o |
| MTA-60 | $D_7$ | o | o | o |
| MTA-90 | $D_8$ | $W_8$ | $R_8$ | $A_8$ |

# FIG.— 12

EP 0 319 551 B1